# EUROPEAN PATENT SPECIFICATION

(11) **EP 1 390 523 B1**
(45) Date of publication and mention of the grant of the patent: **09.05.2007**
(21) Application number: 01989101.9
(22) Date of filing: 16.11.2001
(51) Int. Cl.: G01N 33/574, G01N 33/543, C12Q 1/68

(54) **METHODS AND DEVICES FOR THE QUANTITATIVE DETECTION OF PROSTATE SPECIFIC MEMBRANE ANTIGEN AND OTHER PROSTATIC MARKERS**
VERFAHREN UND VORRICHTUNGEN ZUM QUANTITATIVEN NACHWEIS EINES PROSTATA-SPEZIFISCHEN MEMBRANANTIGENS UND ANDERER PROSTATAMARKER
PROCEDES ET DISPOSITIFS PERMETTANT LA DETECTION QUANTITATIVE DES ANTIGENES DE MEMBRANE SPECIFIQUES DE LA PROSTATE (PSMA) ET D'AUTRES MARQUEURS PROSTATIQUES

(30) Priority: 20.11.2000 US 252452 P
(43) Date of publication of application: 25.02.2004
(73) Proprietor: Eastern Virginia Medical School, Norfolk, VA 23507 (US)
(72) Inventor: WRIGHT, George, L., Jr., Virginia Beach, VA 23455 (US)
(74) Representative: Kiddle, Simon John
(86) International application number: PCT/US2001/043424
(87) International publication number: WO 2002/046448

(56) References cited:
- WO-A-00/50457
- WO-A-01/71360
- WO-A-98/40513
- WO-A1-94/09820
- WRIGHT G L ET AL: "PROTEINCHIP SURFACE ENHANCED LASER DESORPTION/IONIZATION (SELDI) MASS SPECTROMETRY: A NOVEL PROTEIN BIOCHIP TECHNOLOGY FOR DETECTION OF PROSTATE CANCER BIOMARKERS IN COMPLEX PROTEIN MIXTURES" PROSTATE CANCER AND PROSTATIC DISEASES, STOCKTON PRESS, BASINGSTOKE,, GB, vol. 5/6, no. 2, 1999, pages 264-276, XP008001602 ISSN: 1365-7852
- XIAO Z ET AL: "Generation of a Baculovirus Recombinant Prostate-Specific Membrane Antigen and Its Use in the Development of a Novel Protein Biochip Quantitative Immunoassay" PROTEIN EXPRESSION AND PURIFICATION, ACADEMIC PRESS, SAN DIEGO, CA, US, vol. 19, no. 1, June 2000 (2000-06), pages 12-21, XP004435510 ISSN: 1046-5928
- MERCHANT M ET AL: "RECENT ADVANCEMENTS IN SURFACE-ENHANCED LASER DESORPTION/IONIZATION-TIME OF FLIGHT-MASS SPECTROMETRY" ELECTROPHORESIS, WEINHEIM, DE, vol. 21, 2000, pages 1164-1177, XP001079059 ISSN: 0173-0835
- NELSON ET AL.: 'Mass spectrometric immunoassay' ANALYTICAL CHEMISTRY vol. 67, 1995, pages 1153 - 1158, XP001031932

## Description

### BACKGROUND OF THE INVENTION

Prostate carcinoma is the most common noncutaneous cancer in men. Effective treatment options generally exist for non-metastatic forms of prostate cancer, unlike non-organ-confined forms of the disease, especially if androgen deprivation therapy fails (Crawford *et al.,* (1989) "A controlled trial of leuprolide with and without flutamide in prostatic carcinoma," *N. Engl. J. Med* 321:419-424 and Lepor *et al.,* (1982) "The influence of hormonal therapy on survival of men with advanced prostatic cancer," *J. Urol.* 128:335-340). Early diagnosis of the disease is therefore imperative.

Certain prostate cancer screens in current use are highly invasive and generally lack sufficient sensitivity to yield early detection. For example, during digital rectal examinations, physicians attempt to discover prostate gland abnormalities, such as lumpy or enlarged prostate glands, by feeling the gland through the wall of the rectum. Cystoscopy is another common invasive prostate cancer diagnostic technique that also typically lacks adequate sensitivity to detect early prostatic cancers.

Less invasive prostate cancer diagnostic approaches include detecting the differential presence of markers, such as the prostate specific antigen (PSA) biomarker in various body fluids. The effectiveness of a diagnostic assay generally depends upon its specificity and selectivity. Methods of increasing the percentage of true positive and true negative diagnoses for any condition are desirable medical objectives. In the case of prostate cancer, the present diagnostic tests are not completely satisfactory, in that they provide significant numbers of false positive and false negative results. For example, although widely considered to be one of the best cancer markers presently available, PSA-based assays do not always correctly differentiate benign (*e.g.,* benign prostate hyperplasia (BPH)) from malignant prostate disease, and can yield false negative diagnoses of some significant prostate cancers (Osterling (1991) "Prostate specific antigen: a critical assessment of the most useful tumor marker for adenocarcinoma of the prostate," *J. Urol.* 145:907-923 and Pannek and Partin (1998) "The role of PSA and percent of free PSA for staging and prognosis prediction in clinically localized prostate cancer," *Sem. Urol. Oncol.* 16:100-105). In fact, evidence suggests that no single marker will be effective in improving detection, diagnosis and prognosis.

The differential presence of other markers, such as the prostate specific membrane antigen (PSMA), *e.g.*, in primary and metastatic prostate carcinomas indicates that this marker, detected either alone or in combination with other markers (*e.g*., PSA) can be utilized to diagnosis prostate cancer. PSMA is a 750-amino acid, 100 kDa transmembrane glycoprotein (Israeli *et al.,* (1993) "Molecular cloning of a complementary DNA encoding a prostate-specific membrane antigen," *Cancer Res.* 53:227-30). For example, high PSMA expression has been detected in prostate tissues, with weaker expression detected in, *e.g*., the brain, salivary glands, small intestine, and circulating tumor cells via, *e.g*., immunohistochemistry, Western blotting, and RT-PCR. *See, e.g.,* Israeli *et al.,* (1994) "Expression of the prostate-specific membrane antigen," *Cancer Res.* 54:1807-1811, Israeli *et al.,* (1994) "Sensitive nested reverse transcription polymerase chain reaction detection of circulating prostatic tumor cells: comparison of prostate-specific membrane antigen and prostate specific antigen-based assays," *Cancer Res.* 54:6306-6310, and Wright *et al.,* (1995) "Expression of prostate-specific membrane antigen in normal, benign and malignant prostate tissues," *Urologic Oncology* 1:18-28. PSMA has also been detected in various samples using an enzyme-linked immunosorbent assay (ELISA). *See,* Sokoloff *et al.,* (2000) "A dual-monoclonal sandwich assay for prostate-specific membrane antigen: levels in tissues, seminal fluid and urine," *Prostate* 43:150-157. Furthermore, a truncated species of PSMA, namely, PSMA', which lacks the N-terminal sequence or membrane spanning region has also been identified (Su *et al.,* (1995) "Alternatively spliced variants or prostate-specific membrane antigen RNA: ratio of expression as a potential measurement or progression" *Cancer Res.* 55:1441-1443). However, none of these techniques have been successfully used to reliably quantify PSMA or PSMA' in serum, or to define a clear correlation between PSMA or PSMA' levels and prostate cancer or BPH.
Wright Jr et al (Prostate Cancer and Prostatic Diseases, 2:264-276, 1999) discloses the use of Proteinchip® SELDI mas spectrometry for detecting prostate cancer associated biomarkers.
Xiao et al (Protein Expression and Purification, 19:12-21, 2001) discloses the recombinant generation of his tagged PSMA and its use in a biochip immunoassay.
Merchant & Weinberger (Electrophoresis, 21:1164-1167, 2000) review developments in SELDI technology.
WO98/40513 (Ferrari & Stone) discloses a method of detecting prostate caner metastasis by detecting mRNA for PSA or PSMA.

Efforts to determine the clinical utility of PSMA and PSMA' as diagnostic or prognostic biomarkers have been hampered by the lack of a sensitive immunoassay for quantification of PSMA and PSMA' in body fluids. The present invention offers a reliable system to quantify PSMA and/or PSMA' proteins or encoding nucleic acids and to address their potential as a diagnostic/prognostic marker for prostate cancer and BPH. The invention further provides an innovative platform to rapidly and simultaneously detect and measure other markers in addition to PSMA and/or PSMA'. These and other features will become apparent upon complete review of the following disclosure.

### SUMMARY OF THE INVENTION

Proteins, such as PSMA and PSMA', and/or nucleic acids encoding those proteins (*e.g*., mRNAs) have been discovered to function as markers in prostate cancer (CaP) or benign prostate hyperplasia versus a negative diagnosis. Compared to a negative diagnosis, the markers are, variously, more frequently detected, less frequently detected, or differentially detected. The measurement of these markers, alone or in combination, in patient samples, provides information that the diagnostician can correlate with probable diagnoses of prostate cancer, benign prostate hyperplasia or with a negative diagnosis (*e.g.*, normal or disease-free). More specifically, an amount of PSMA/PSMA' above the normal range is positively correlated with a diagnosis of prostate cancer, and an amount of PSMA/PSMA' below the normal range is positively correlated with benign prostate hyperplasia or prostatitis. The markers are characterized by molecular weight and can be resolved from other sample components, such as other proteins by, *e.g.,* chromatographic separation coupled with mass spectrometry. In preferred embodiments, the method of resolution involves Surface Enhanced Laser Desorption/Ionization (SELDI) mass spectrometry, in which the surface of the mass spectrometry probe plays an active role in the presentation of the analyte to an energy source for desorption and ionization of the analyte. Affinity capture surface enhanced LDI-based immunoassays provide significant advantages to the development of clinical diagnostic assays, including the capacity to quantify PSMA, PSMA', other protein-based prostatic markers in addition to associated nucleic acids, and the ability to simultaneously detect and measure multiple PSMA isoforms and other prostatic markers.

In one aspect, the present invention relates to methods for aiding in a differential diagnosis of prostate cancer, benign hyperplasia or a negative diagnosis as set out in the claims.
In one embodiment, the present invention provides method for aiding in a differential diagnosis of prostate cancer, benign hyperplasia, or a negative diagnosis, the method comprising:
(a) exposing a sample from a subject to at least one substrate-bound adsorbent that captures PSMA and/or PSMA', thereby capturing the PSMA and/or the PSMA' in the sample;
(b) detecting the captured PSMA and/or the captured PSMA; by gas phase ion spectrometry; and
(c) correlating the detected PSMA and/or the detected PSMA' with a probable diagnosis of prostate cancer, benign prostate hyperplasia, or a negative diagnosis; wherein an amount of the PSMA and/or the PSMA' above a control amount is positively correlated with a positive diagnosis of prostate cancer and an amount of the PSMA and/or the PSMA' below a control amount is positively correlated with a positive diagnosis of benign prostate hyperplasia. The method includes fractionating a test sample and isolating a sample comprising PSMA/PSMA' and detecting the PSMA/PSMA' from the sample using gas phase ion spectrometry. In one embodiment, the method comprises (a) exposing the sample to a substrate-bound adsorbent that captures the PSMA or PSMA' in the sample, and (b) quantifying different types of samples are optionally used, preferred samples include serum and cell lysate (*e.g.*, derived from prostatic tissue or cells).

In one embodiment, the method further includes fractionating biomolecules in the sample to collect a sample fraction that includes the PSMA or PSMA' in which the sample fraction is used as the sample in (a). For example, the biomolecules are optionally fractionated by, *e.g.*, electrophoresis, dialysis, filtration, centrifugation, or the like. In other embodiments, the biomolecules are fractionated by, *e.g.,* high performance liquid chromatography, affinity chromatography, ion exchange chromatography, size exclusion chromatography, or the like. In another embodiment, the biomolecules are fractionated by (i) separating biomolecules in the sample into a one- or two-dimensional array of spots, wherein each spot comprises one or more of the biomolecules, and (ii) selecting and removing a spot from the array which is suspected of comprising the PSMA or PSMA'. Optionally, the method additionally includes digesting the biomolecules in the selected spot with an enzyme prior to analyzing the selected spot by the gas phase ion spectrometry. Moreover, the method typically includes comparing an amount of detected or quantified PSMA or PSMA' with a control.

The method for quantifying PSMA or PSMA' also includes other sample preparation or pre-fractionation techniques. For example, (a) optionally further includes removing material other than the captured PSMA or PSMA', *e.g.,* prior to exposing the sample to the adsorbent. In this embodiment, the material is typically removed by one or more washes in which each of the washes includes an identical or a different elution condition relative to a preceding wash. For example, elution conditions generally differ according to pH, buffering capacity, ionic strength, a water structure characteristic, detergent type, detergent strength, hydrophobicity, dielectric constant, concentration of a solute, or the like.

The invention utilizes various types of substrate-bound adsorbents to capture targeted prostatic markers, such as PSMA or PSMA'. Optionally, the substrate-bound adsorbent is provided as a biochip that includes a substrate with at least one surface feature having the substrate-bound adsorbent bound to the substrate.
In certain embodiments, the substrate-bound adsorbent includes a monoclonal antibody that specifically captures the PSMA or PSMA'. In other embodiments, the substrate-bound adsorbent includes a protein that specifically binds an immunoglobulin. In these embodiments, the method includes exposing the sample to the immunoglobulin in which the immunoglobulin specifically binds PSMA or PSMA' to form a PSMA- or PSMA'-comple.x, and exposing the complex to the substrate-bound adsorbent. Alternatively, the substrate-bound adsorbent includes a bead or resin derivatized with the adsorbent.

In certain embodiments, the method includes quantifying both the PSMA and the PSMA' in the sample. When both PSMA and PSMA' are quantified in a sample, the method also typically includes comparing amounts of quantified PSMA and PSMA' with one another or with a control. Optionally, the method includes comparing a ratio of amounts of quantified PSMA and PSMA' with a control.

In other embodiments, the method additionally includes quantifying at least one other prostatic marker in the sample in which (a) also includes exposing the sample to at least one substrate-bound adsorbent that captures the other prostatic marker. Optionally, the substrate-bound adsorbent is provided as a biochip that includes a substrate with at least one surface feature including the substrate-bound adsorbent bound to the substrate and in which prostatic markers are captured on the at least one surface feature. In other embodiments, the substrate-bound adsorbent includes a protein that specifically binds immunoglobulins. In these embodiments, the method includes exposing the sample to the immunoglobulins, each of which specifically binds one of the prostatic biomarkers to form complexes with the prostatic markers, and exposing the complexes to the substrate-bound adsorbent. The other prostatic marker typically includes, *e.g.*, PSMA, PSMA', prostate-specific antigen (PSA), free-PSA, complexed-PSA, prostatic acid phosphatase (PAP), prostate specific peptide 94 (PSP94), prostate stem cell antigen (PSCA), or the like. In this embodiment, the method also generally includes comparing amounts of quantified PSMA or PSMA' and the other prostatic marker with one another or with a control. Alternatively, the method further includes comparing a ratio of amounts of quantified PSMA or PSMA' and the other prostatic marker with a control.

In preferred embodiments, the gas phase ion spectrometry is laser desorption/ionization mass spectrometry, *e.g.,* surface enhanced laser desorption/ionization mass spectrometry. For example, the method typically includes (i) generating data on the sample with a mass spectrometer indicating intensity of signal for one or more mass/charge ratios, (ii) transforming the data into computer-readable form, and (iii) operating a programmable digital computer and executing an algorithm that detects signal in the computer-readable data representing the PSMA or PSMA'.

In one embodiment, the laser desorption/ionization mass spectrometry includes (i) providing a probe adapted for use with a mass spectrometer comprising an adsorbent attached thereto, (ii) contacting the sample with the adsorbent, and (iii) desorbing and ionizing the PSMA or PSMA' from the probe and detecting the desorbed/ionized PSMA or PSMA' with the mass spectrometer. Optionally, the substrate includes a probe adapted for use with the mass spectrometer, or the substrate is suitable for being placed on a probe which is adapted for use with the mass spectrometer.

In another embodiment, the laser desorption/ionization mass spectrometry includes (i) providing a substrate comprising an adsorbent attached thereto, (ii) contacting the sample with the adsorbent, (iii) placing the substrate on a probe adapted for use with a mass spectrometer comprising an adsorbent attached thereto, and (iv) desorbing and ionizing the PSMA or PSMA' from the probe and detecting the desorbed/ionized PSMA or PSMA' with the mass spectrometer. For example, the substrate typically includes a bead or resin derivatized with the adsorbent.

The correlation step in the method generally takes into account a presence or absence of the PSMA or PSMA' in the sample and a frequency of PSMA or PSMA' detection in a control. In addition, the correlation also typically takes into account a quantity of the PSMA or PSMA' in the sample relative to a control quantity of the PSMA or PSMA'. For example, an amount of PSMA or PSMA' above a control amount is positively correlated with a positive diagnosis of prostate cancer and an amount of PSMA or PSMA' below a control amount is positively correlated with a positive diagnosis of benign prostate hyperplasia. In other embodiments, an immunoassay is used for detecting the PSMA or PSMA' in the sample.

In certain embodiments, the method includes detecting the PSMA or PSMA' by gas phase ion spectrometry. In preferred embodiments, the gas phase ion spectrometry is laser desorption/ionization mass spectrometry, e.g., surface enhanced laser desorption/ionization mass spectrometry. The method typically includes (i) generating data on the sample with a mass spectrometer indicating intensity of signal for one or more mass/charge ratios, (ii) transforming the data into computer-readable form, and (iii) operating a programmable digital computer and executing an algorithm that determines closeness-of-fit between the computer-readable data and data indicating a diagnosis of prostate cancer, benign prostate hyperplasia, or a negative diagnosis. The algorithm optionally includes an artificial intelligence algorithm or a heuristic learning algorithm. For example, the artificial intelligence algorithm typically includes a fuzzy logic instruction set, a cluster analysis instruction set, a neural network, a genetic algorithm, or the like.

In one aspect, the laser desorption/ionization mass spectrometry includes (i) providing a substrate comprising at least one adsorbent attached thereto, (ii) contacting the sample with the adsorbent, and (iii) desorbing and ionizing the PSMA or PSMA' from the substrate and detecting the desorbed/ionized PSMA or PSMA' with the mass spectrometer. In one embodiment, the substrate is a probe adapted for use with the mass spectrometer. In another embodiment, the substrate is suitable for being placed on a probe which is adapted for use with the mass spectrometer, e.g., a bead or resin derivatized with the adsorbent. Various chromatographic or biomolecular interaction adsorbents are optionally utilized.

In one embodiment, the method includes detecting and correlating both the PSMA and the PSMA' in the sample. A detected presence of the PSMA and the PSMA' is typically correlated with, e.g., a positive diagnosis of prostate cancer or benign prostate hyperplasia. In another embodiment, the correlation takes into account a presence or absence of the PSMA, and the PSMA' in the sample and a frequency of PSMA and PSMA' detection in a control. In this embodiment, the correlation generally also takes into account quantities of the PSMA and the PSMA', or a ratio thereof, in the sample relative control quantities of the PSMA and the PSMA', or a ratio thereof.

In another embodiment, the method includes detecting and correlating at least one other prostatic marker in the sample. The other prostatic marker typically includes, *e.g.,* PSMA, PSMA', PSA, free-PSA, complexed-PSA, PAP, PSP94, PSCA, or the like. For example, a detected presence of the PSMA or PSMA' and the other prostatic marker is typically correlated with, e.g., a positive diagnosis of prostate cancer or benign prostate hyperplasia. In one aspect, the correlation takes into account a presence or absence of the PSMA or PSMA' and the other prostatic marker in the sample and a frequency of detection of the PSMA or PSMA' and the other prostatic marker in a control. The correlation also optionally takes into account quantities or ratios of quantities of the PSMA or PSMA' and the other prostatic marker in the sample relative to control quantities or ratios of quantities of the PSMA or PSMA' and the other prostatic marker.

The methods may employ a biochip that is removably insertable into a gas phase ion spectrometer that includes a substrate with at least one surface feature having at least one adsorbent bound to the substrate in which the adsorbent is capable of capturing PSMA or PSMA'. The adsorbent is typically capable of resolving PSMA or PSMA' from a sample under at least one elution condition. Various chromatographic or biomolecular interaction adsorbents are optionally utilized. In one embodiment, the at least one surface feature of the biochip includes a plurality of surface features. For example, the plurality of surface features is arranged in a line, an orthogonal array, a circle, or an n-sided polygon, wherein n is three or greater. The plurality of surface features typically includes a logical or spatial array. Optionally, each of the plurality of surface features comprises identical or different adsorbents, or one or more combinations thereof. For example, at least two of the plurality of surface features optionally include identical or different adsorbents, or one or more combinations thereof. In certain embodiments, the biochip further includes at least one other adsorbent in addition to the adsorbent capable of capturing PSMA or PSMA' in which the other adsorbent is bound to the substrate at one or more of the plurality of surface features. The other adsorbent is typically capable of capturing at least one other prostatic marker, such as PSMA, PSMA', PSA, free-PSA, complexed-PSA, PAP, PSP94, PSCA, or the like.

The methods may be carried out using a kit that includes (a) at least one adsorbent that captures PSMA or PSMA', (b) a set of instructions for capturing PSMA or PSMA' from a sample by exposing the sample to the adsorbent and quantifying the captured PSMA or PSMA' by gas phase ion spectrometry, and (c) at least one container for packaging the adsorbent and the set of instructions. Optionally, the kit also includes at least one eluant for washing the adsorbent to remove material other than the captured PSMA or PSMA'. The adsorbent typically includes a solid phase adsorbent. In one embodiment, the solid phase adsorbent is provided as a biochip that includes a substrate with at least one surface feature having the solid phase adsorbent bound to the substrate. The substrate is generally a probe adapted for use with a gas phase ion spectrometer. The kit optionally includes the probe. In certain embodiments, the probe includes a substrate with a plurality of surface features. For example, each of the plurality of surface features optionally includes one or more adsorbents bound to the substrate. In other embodiments, the solid phase adsorbent includes a bead or resin derivatized with the adsorbent. For example, the bead or resin derivatized with the at least one adsorbent is typically suitable for being placed on a probe adapted for use with a gas phase ion spectrometer. The kit also optionally includes the probe. As an additional option, the kit also includes at least one reference or control.

The methods may also employ a device or integrated system for quantifying PSMA or PSMA' in at least one sample that includes (a) at least one adsorbent capable of capturing the PSMA or PSMA' in the sample, and (b) a gas phase ion spectrometer for quantifying the PSMA or PSMA' captured on the adsorbent. The gas phase ion spectrometer is optionally a laser desorption/ionization mass spectrometer. The device or integrated system also typically includes a computer or a computer readable medium, operably connected to the gas phase ion spectrometer, that includes a computer program having one or more of, *e.g.,* (i) at least one instruction set for analyzing or processing data quantified by the gas phase ion spectrometer, (ii) at least one instruction set for entering data into a database, or, (iii) at least one instruction set for determining correlations between at least one quantified prostatic marker, or combinations of quantified prostatic markers, and a diagnosis of prostate cancer, benign prostate hyperplasia, or a negative diagnosis.

The present invention also provides a method of aiding in a differential diagnosis of prostrate cancer, benign prostate hyperplasia, or a negative diagnosis the method comprising:
(a) exposing a sample from a subject to at least one adsorbent that captures a prostatic marker mRNA, thereby capturing the prostatic marker mRNA in the sample;
(b) detecting the captured prostatic marker mRNA by gas phase ion spectrometry; and,
(c) correlating the detected prostatic marker mRNA with a probable diagnosis of prostate cancer, benign prostate hyperplasia, or a negative diagnosis.
   The sample includes, *e.g.*, cell lysate derived from prostatic tissue or cells, while the adsorbent (*e.g*., a biomolecular interaction adsorbent) generally includes, *e.g.*, prostatic marker cDNA that corresponds to the prostatic marker mRNA. In preferred embodiments, the gas phase ion spectrometry is laser desorption/ionization mass spectrometry. Additionally, the method also typically includes comparing a quantity of the captured prostatic marker mRNA with a control. Optionally, the method includes quantifying a plurality of different prostatic marker mRNAs.

The, method may also be carried out using a biochip or kit for the detection and quantification of prostatic marker mRNAs. The biochip includes a substrate with at least one surface feature comprising at least one adsorbent bound to the substrate in which the adsorbent is capable of capturing one or more prostatic marker mRNAs. The kit includes (a) at least one adsorbent that captures a prostatic marker mRNA, (b) a set of instructions for capturing the prostatic marker mRNA from a sample by exposing the sample to the adsorbent and quantifying the captured prostatic marker mRNA by gas phase ion spectrometry, and (c) at least one container for packaging the adsorbent and the set of instructions.

### BRIEF DESCRIPTION OF THE DRAWINGS

Figure 1 schematically depicts a surface enhanced laser desoiption/ionization time-of-flight (TOF) mass spectrometry (MS) system configured for surface enhanced laser desorption/ionization.

Figure 2 schematically shows a PSMA surface enhanced laser desorption/ionization MS immunoassay.

Figure 3 schematically illustrates the surface enhanced laser desorption/ionization-TOF-MS technology.

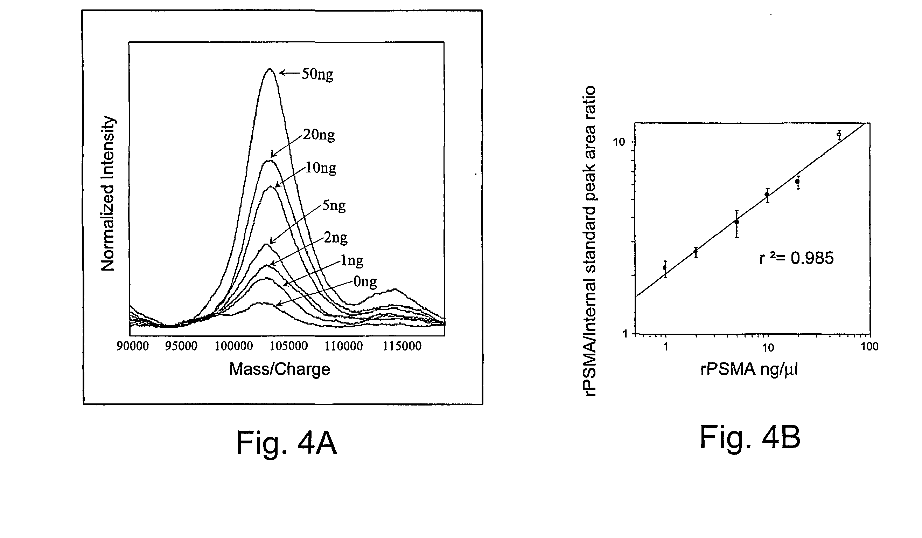

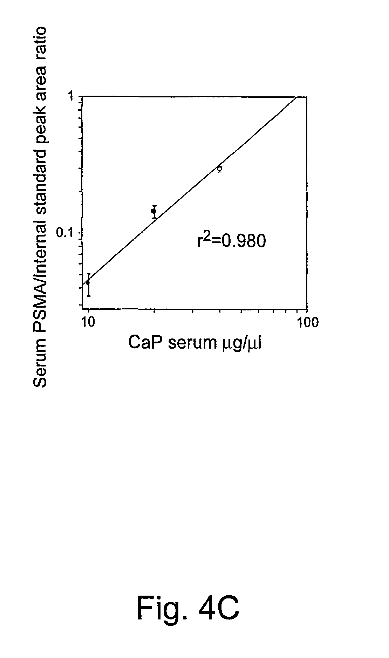

Figures 4A-C show a surface enhanced laser desorption/ionization MS immunoassay for quantification of PSMA in serum. Different concentrations of rPSMA or serum were applied to preactivated PS-1 ProteinChip® arrays containing bound MoAb 7E11, and the chips were washed and subjected to surface enhanced laser desorption/ionization mass analysis. Figure 4A show the spectra of the different rPSMA concentrations (at 1 -50ng/µl) showing that the increases in both intensity and peak areas correlated with increasing protein concentrations. Figure 4B shows an rPSMA standard curve. Recombinant PSMA signal intensity of each spectrum shown in Figure 4A was normalized to the internal standard (β-galactoglobulin, 25 fmol/µl). A linear curve was generated by plotting the peak ratio (rPSMA/internal standard) versus rPSMA protein concentration. The mean and standard deviation of three separate mass spectra are shown. Figure 4C shows PSMA detected in a serum sample from a patient diagnosed with prostate cancer (PCA). The analysis was the same as used for generating the rPSMA standard curve, except that 100 fmol/µl β-galactoglobulin was spiked as the internal standard for normalization. Doubling dilutions (i.e., 10, 20, and 40 µg/µl) of total serum proteins show a linear regression. The mean and standard deviation of three separate mass spectra are shown.

Figure 5 is a chart showing the levels of serum PSMA determined by affinity capture surface enhanced laser desorption/ionization immunoassay in samples taken from patients with each of prostatitis, prostate cancer (PCA), benign prostate hyperplasia (BPH), and a normal or negative diagnosis. The patients with negative diagnoses are further divided into two age-based sub-populations of males less than age 50 (<50) and males greater than age 50 (>50). The bar shown for each patient population indicates the mean PSMA level for that population.

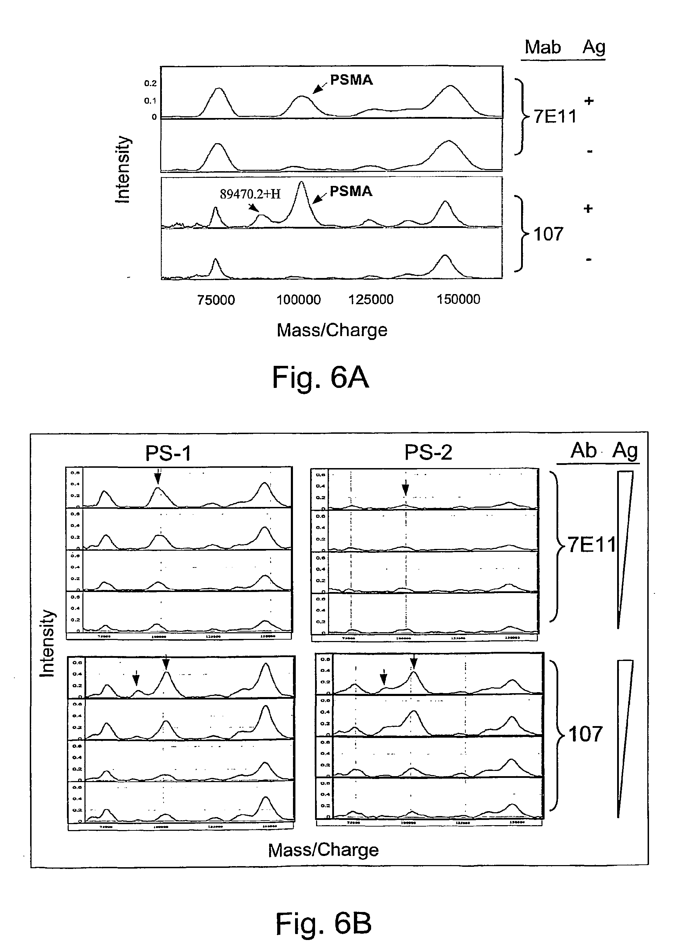

Figures 6A and B are mass spectral traces showing the detection of PSMA isoforms by affinity capture surface enhanced laser desorption/ionization immunoassay in whole cell lysate from the human androgen-dependent prostate carcinoma cell line (LNCaP). Figure 6A shows the spectra obtained using 7E11 or 107 antibodies to capture PSMA in LNCaP whole cell lysate (Ag) +: LNCaP whole cell lysate added; -: dilution buffer only (0.1% Triton X-100 in PBS). Figure 6B shows a comparison of the standard curves of PSMA isoforms on PS-1 and PS-2 surfaces. The wedges indicate the relative amount of LNCaP whole cell lysate used (from 6 ng/µl, 12 ng/µl, 25 ng/µl to 50 ng/µl). The arrows at the higher mass/charge ratios show the 100kD PSMA isoform, while the arrows at the low mass/charge ratios show the 89kD PSMA' isoform.

Figures 7A-H show a multiplex immunoassay for detection of PSMA and PSA in seminal plasma.

### DEFINITIONS

Unless defined otherwise, all technical and scientific terms used herein have the meaning commonly understood by a person skilled in the art to which this invention belongs. The following references provide one of skill with a general definition of many of the terms used in this invention: Singleton *et al., Dictionary of Microbiology and Molecular Biology* (2nd ed. 1994); *The Cambridge Dictionary of Science and Technology* (Walker ed., 1988); *The Glossary of Genetics,* 5th Ed., R. *Rieger et al.* (eds.), Springer Verlag (1991); and Hale & Marham, *The Harper Collins Dictionary of Biology* (1991). As used herein, the following terms have the meanings ascribed to them unless specified otherwise.

"Prostate cancer" refers to an uncontrolled (malignant) growth of cells in the male prostate gland. The prostate gland surrounds the urethra at the base of the urinary bladder and is responsible for helping to control urination as well as forming some of the components of seminal fluid. Prostate cancers are typically classified or staged based on their aggressiveness and the degree that they are differentiated from the surrounding prostate tissue. There are several different ways to stage tumors, including the Whitmore-Jewett system, the T classification and the Gleason score. *See, e.g.,* Zagars *et al.,* (1994) "The T classification of clinically localized prostate cancer. An appraisal based on disease outcome after radiation therapy," *Cancer* 73(7):1904-1912. To illustrate, according to the Whitmore-Jewett system, prostate cancers are staged using an A-B-C-D staging system. This staging system contains several substages, but it basically categorizes tumors using the following scale: (A) tumor not palpable but is detectable in microscopic biopsy, (B) palpable tumor confined to prostate, (C) extension of tumor beyond prostate with no distant metastasis, and (D) cancer has spread to regional lymph nodes. Prostate cancers typically spread by extending into the seminal vesicles, bladder, and peritoneal cavity and metastasize to the lymph nodes, bones, lungs, liver, and kidneys. Treatment options for prostate cancer generally include radiation therapy, surgery, hormonal therapy and chemotherapy

"Benign prostate hyperplasia" or "BPH" refers to a nonmalignant (noncancerous) enlargement of the prostate gland. It is also known as benign prostatic hypertrophy and as nodular hyperplasia of the prostate. In BPH the normal elements of the prostate gland grow in size and number. Their sheer bulk may compress the urethra and impede the flow of urine from the bladder. This leads to urine retention and the need for frequent urination. In severe cases, complete blockage can occur. Medical therapy of BPH includes drugs such as finasteride and terazosin. Prostate enlargement in BPH is directly dependent on dihydrotestosterone (DHT), the principal androgen hormone in the prostate. Finasteride (PROSCAR®) blocks the enzyme needed to make DHT and so lowers blood and tissue DHT levels and helps reduce the size of the prostate. Terazosin (HYTRIN®) belongs to a class of medications called alpha 1 blockers which relaxes the smooth muscles of the arteries, the prostate, and the bladder neck. Relaxing the smooth muscles around the bladder neck helps to relieve urinary obstruction caused by an enlarged prostate in BPH.

"Serum" or "blood serum" refers to the watery portion of a bodily fluid (*e.g*., from blood) that remains after coagulation. For example, it is typically a clear yellowish fluid that remains from blood plasma after fibrinogen, prothrombin, and other clotting factors have been removed by, *e.g.,* clot formation.

"Marker" or "biomarker" in the context of the present invention refers to an organic biomolecule, e.g., a polypeptide or a nucleic acid (*e.g*., an mRNA or the like) which is differentially present in a sample taken from patients having prostate cancer or benign prostate hyperplasia as compared to a comparable sample taken from control subjects, such as persons with negative diagnoses or undetectable cancer (*e.g.*, normal or healthy subjects). For example, a marker can be a polypeptide or a nucleic acid (*e.g.*, having a particular apparent molecular weight) which is present at an elevated or decreased level in samples of prostate cancer patients compared to samples of patients with negative diagnoses.

The phrase "differentially present" refers to differences in the quantity and/or the frequency of a marker, *e.g.*, a polypeptide or a nucleic acid *(e.g.,* of a particular apparent molecular weight), present in a sample taken from a patient having prostate cancer as compared to a comparable sample taken from a patient that does not have prostate cancer (e.g., has benign prostate hyperplasia or a negative diagnosis). For example, a marker can be a polypeptide or a nucleic acid (*e.g.,* an mRNA or the like), which is present at an elevated level or at a decreased level in samples from prostate cancer or BPH patients compared to samples from control subjects. Alternatively, a marker can be a polypeptide or a nucleic acid that is detected at a higher frequency or at a lower frequency in samples from prostate cancer or BPH patients compared to samples from control subjects. A marker can be differentially present in terms of quantity, frequency or both.

A biomarker, e.g., a polypeptide or nucleic acid is differentially present between two samples (*e.g.*, two sets of samples) if the frequency of detecting the polypeptide or the nucleic acid in one sample is statistically significantly different (higher or lower) than the frequency of detection of the polypeptide or the nucleic acid in the other sample (or other set of samples) and/or a control sample. For example, two sets of data can be compared using student's t-test, and P<0.05 can be considered statistically significant. In another example, a polypeptide or a nucleic acid is differentially present between the two sets of samples if it is detected at least about 120%, at least about 130%, at least about 150%, at least about 180%, at least about 200%, at least about 300%, at least about 500%, at least about 700%, at least about 900%, or at least about 1000% more frequently, or less frequently, in one set of samples than the other set of samples.

Alternatively or additionally, a polypeptide or nucleic acid is differentially present between the two samples if the amount of the polypeptide or nucleic acid in one sample is statistically significantly different from the amount of the polypeptide or nucleic acid in the other sample. For example, a polypeptide or nucleic acid is differentially present between the two samples if it is present at least about 120%, at least about 130%, at least about 150%, at least about 180%, at least about 200%, at least about 300%, at least about 500%, at least about 700%, at least about 900%, or at least about 1000% greater than it is present in the other sample, or if it is detectable in one sample and not detectable in the other. Any polypeptides or nucleic acids that are differentially present in samples taken from prostate cancer patients as compared to subjects who do not have prostate cancer (*e.g.*, benign prostate hyperplasia patients) can be used as markers.

"Diagnostic" means identifying the presence or nature of a pathologic condition. Diagnostic methods differ in their sensitivity and specificity. The "sensitivity" of a diagnostic assay is the percentage of diseased individuals who test positive (percent of "true positives"). Diseased individuals not detected by the assay are "false negatives." Subjects who are not diseased and who test negative in the assay, are termed "true negatives." The "specificity" of a diagnostic assay is 1 minus the false positive rate, where the "false positive" rate is defined as the proportion of those without the disease who test positive. While a particular diagnostic method may not provide a definitive diagnosis of a condition, it suffices if the method provides a positive indication that aids in diagnosis.

A "test amount" of a marker refers to an amount of a marker present in a sample being tested. A test amount can be either in absolute amount (e.g., µg/ml) or a relative amount (*e.g*., relative intensity of signals).

A "diagnostic amount" of a marker refers to an amount of a marker in a subject's sample that is consistent with a diagnosis of prostate cancer or benign prostate hyperplasia. A diagnostic amount can be either in absolute amount *(e.g.,* µg/ml) or a relative amount (*e.g*., relative intensity of signals).

A "control amount" of a marker can be any amount or a range of amount which is to be compared against a test amount of a marker. For example, a control amount of a marker can be the amount of a marker (*e.g.*, PSMA, PSMA', PSMA mRNA, or the like) in a prostate cancer patient, a BPH patient or a person without prostate cancer or BPH. A control amount can be either in absolute amount *(e.g.,* µg/ml) or a relative amount (*e.g.,* relative intensity of signals).

"Probe" refers to a device that is removably insertable into a gas phase ion spectrometer and comprises a substrate having a surface, or one or more surface features, for presenting markers for detection. A probe can comprise a single substrate or a plurality of substrates. Terms such as ProteinChip® array, biochip, or chip are also used herein to refer to specific kinds of probes.

"Substrate" or "probe substrate" refers to a solid phase onto which an adsorbent can be provided (*e.g.,* by attachment, deposition, or the like). "Surface feature" refers to a particular portion, section, or area of a substrate or probe substrate onto which adsorbent can be provided.

"Adsorbent" refers to any material capable of adsorbing a marker. The term "adsorbent" is used herein to refer both to a single material ("monoplex adsorbent") (*e.g.*, a compound or functional group) to which the marker is exposed, and to a plurality of different materials ("multiplex adsorbent") to which the marker is exposed. The adsorbent materials in a multiplex adsorbent are referred to as "adsorbent species." For example, a surface feature on a probe substrate can comprise a multiplex adsorbent characterized by many different adsorbent species (*e.g.,* ion exchange materials, metal chelators, antibodies, cDNAs, or the like), having different binding characteristics. Substrate material itself can also contribute to adsorbing a marker and may be considered part of an "adsorbent." A "biomolecular interaction adsorbent" or "biospecific adsorbent," such as an affinity adsorbent, a polypeptide, an enzyme, a prostatic marker substrate, a receptor, an antibody *(e.g.*, a monoclonal antibody, *etc*.), or the like, typically has higher specificity for a target marker than a "chromatographic adsorbent," which includes, *e.g.,* an anionic adsorbent, a cationic adsorbent, a hydrophobic interaction adsorbent, a hydrophilic interaction adsorbent, a metal-chelating adsorbent, or the like.

"Adsorption," "capture" or "retention" refers to the detectable binding between an absorbent and a marker either before or after washing with an eluant (selectivity threshold modifier) or a washing solution.

"Eluant" or "washing solution" refers to an agent that can be used to mediate adsorption of a marker to an adsorbent. Eluants and washing solutions also are referred to as "selectivity threshold modifiers." Eluants and washing solutions can be used to wash and remove unbound materials from the probe substrate surface.

"Resolve," "resolution," or "resolution of marker" refers to the detection of at least one marker in a sample. Resolution includes the detection of a plurality of markers in a sample by separation and subsequent differential detection. Resolution does not require the complete separation of a marker from all other markers in a mixture. Rather, any separation that allows the distinction between at least two markers suffices.

"Gas phase ion spectrometer" refers to an apparatus that measures a parameter which can be translated into mass-to-charge ratios of ions formed when a sample is volatilized and ionized. Generally ions of interest bear a single charge, and mass-to-charge ratios are often simply referred to as mass. Gas phase ion spectrometers include, for example, mass spectrometers, ion mobility spectrometers, and total ion current measuring devices.

"Mass spectrometer" refers to a gas phase ion spectrometer that includes an inlet system, an ionization source, an ion optic assembly, a mass analyzer, and a detector.

"Laser desorption mass spectrometer" refers to a mass spectrometer which uses laser as a means to desorb, volatilize, and ionize an analyte.

"Detect" refers to identifying the presence, absence or amount of the object to be detected.

The terms "polypeptide," "peptide" and "protein" are used interchangeably herein to refer to a polymer of amino acid residues. The terms apply to amino acid polymers in which one or more amino acid residues are analogs, derivatives or mimetics of corresponding naturally occurring amino acids, as well as to naturally occurring amino acid polymers. For example, polypeptides can be modified or derivatized, *e.g.,* by the addition of carbohydrate residues to form glycoproteins. The terms "polypeptide," "peptide" and "protein" include glycoproteins, as well as non-glycoproteins.

"Derivative" refers to a chemical substance related structurally to another substance, or a chemical substance that can be made from another substance (i.e., the substance it is derived from), *e.g.,* through chemical or enzymatic modification.

The term "nucleic acid" refers to DNA (*e.g.,* cDNA or the like) and RNA (*e.g.,* mRNA or the like), which are polymers or oligomers of deoxyribonucleotides and ribonucleotides (or derivatives thereof), respectively. Two nucleic acid sequences "correspond" when they have the same sequence, or when one nucleic acid sequence is a complementary to at least a subsequence of the other. For example, the cDNA produced by, *e.g.,* RT-PCR of the mRNA encoding PSMA, corresponds to that mRNA.

A "prostatic marker cDNA" refers to cDNA that corresponds to an mRNA encoding a prostatic marker, such as PSMA, PSMA', PSA, prostatic acid phosphatase (PAP), prostate specific peptide 94 (PSP94), prostate stem cell antigen (PSCA), or the like. Prostatic marker cDNAs are typically used in the present invention as adsorbents for the detection/quantification of corresponding mRNAs from selected sample sources via gas phase ion spectrometry (*e.g.*, surface enhanced laser desorption/ionization). Prostatic marker cDNAs are typically generated using RT-PCR or the like.

"Detectable moiety" or a "label" refers to a composition detectable by spectroscopic, photochemical, biochemical, immunochemical, or chemical means. For example, useful labels include ³²P, ³⁵S, fluorescent dyes, electron-dense reagents, enzymes (*e.g.*, as commonly used in an ELISA), biotin-streptavadin, dioxigenin, haptens and proteins for which antisera or monoclonal antibodies are available, or nucleic acid molecules with a sequence complementary to a target. The detectable moiety often generates a measurable or detectable signal, such as a radioactive, chromogenic, or fluorescent signal, that can be used to quantify the amount of bound detectable moiety in a sample. The detectable moiety can be incorporated in or attached to a primer or probe either covalently, or through ionic, van der Waals or hydrogen bonds, *e.g.,* incorporation of radioactive nucleotides, or biotinylated nucleotides that are recognized by streptavadin. The detectable moiety may be directly or indirectly detectable. Indirect detection can involve the binding of a second directly or indirectly detectable moiety to the detectable moiety. For example, the detectable moiety can be the ligand of a binding partner, such as biotin, which is a binding partner for streptavadin, or a nucleotide sequence, which is the binding partner for a complementary sequence, to which it can specifically hybridize. The binding partner may itself be directly detectable, for example, an antibody may be itself labeled with a fluorescent molecule. The binding partner also may be indirectly detectable, for example, a nucleic acid having a complementary nucleotide sequence can be a part of a branched DNA molecule that is in turn detectable through hybridization with other labeled nucleic acid molecules. (*See, e.g.,* P. D. Fahrlander and A. Klausner, *Bio*/*Technology* 6:1165 (1988)). Quantification of the signal is achieved by, *e.g.,* scintillation counting, densitometry, or flow cytometry.

"Antibody" refers to a polypeptide ligand substantially encoded by an immunoglobulin gene or immunoglobulin genes, or fragments thereof, which specifically binds and recognizes an epitope (*e.g.,* an antigen). The recognized immunoglobulin genes include the kappa and lambda light chain constant region genes, the alpha, gamma, delta, epsilon and mu heavy chain constant region genes, and the myriad immunoglobulin variable region genes. Antibodies exist, e.g., as intact immunoglobulins or as a number of well characterized fragments produced by digestion with various peptidases. This includes, *e.g*., Fab' and F(ab)'₂ fragments. The term "antibody," as used herein, also includes antibody fragments either produced by the modification of whole antibodies or those synthesized de novo using recombinant DNA methodologies. It also includes polyclonal antibodies, monoclonal antibodies, chimeric antibodies, humanized antibodies, or single chain antibodies. The "Fc" portion of an antibody refers to that portion of an immunoglobulin heavy chain that comprises one or more heavy chain constant region domains, CH1, CH2 and CH3, but does not include the heavy chain variable region.

"Immunoassay" is an assay that uses an antibody to specifically bind an antigen. The immunoassay is characterized by the use of specific binding properties of a particular antibody to isolate, target, and/or quantify the antigen.

The phrase "specifically (or selectively) binds" to an antibody or "specifically (or selectively) immunoreactive with," when referring to a protein or peptide, refers to a binding reaction that is determinative of the presence of the protein in a heterogeneous population of proteins and other biologics. Thus, under designated immunoassay conditions, the specified antibodies bind to a particular protein at least about two times the background and do not substantially bind in a significant amount to other proteins present in the sample. Specific binding to an antibody under such conditions may require an antibody that is selected for its specificity for a particular protein. For example, polyclonal antibodies raised to, *e.g.*, PSMA, PSMA', or the like, from specific species such as rat, mouse, or human can be selected to obtain only those polyclonal antibodies that are specifically immunoreactive with, e.g., PSMA, PSMA', or the like, and not with other proteins, except for polymorphic variants and alleles of seminal basic protein. This selection may be achieved by subtracting out antibodies that cross-react with, *e.g*., PSMA, PSMA', or the like, molecules from other species. A variety of immunoassay formats may be used to select antibodies specifically immunoreactive with a particular protein. For example, solid-phase ELISA immunoassays are routinely used to select antibodies specifically immunoreactive with a protein (*see, e.g.,* Harlow & Lane, *Using Antibodies: A Laboratory Manual* (1998), for a description of immunoassay formats and conditions that can be used to determine specific immunoreactivity). Typically a specific or selective reaction will be at least about twice background signal or noise and more typically more than about 10 to about 100 times background.

"Energy absorbing molecule" or "EAM" refers to a molecule that absorbs energy from an ionization source in a mass spectrometer thereby aiding desorption of analyte, such as a marker, from a probe surface. Depending on the size and nature of the analyte, the energy absorbing molecule can optionally be used.
Energy absorbing molecules used in MALDI are frequently referred to as "matrix." Cinnamic acid derivatives, sinapinic acid ("SPA"), cyano hydroxy cinnamic acid ("CHCA") and dihydroxybenzoic acid are frequently used as energy absorbing molecules in laser desorption of bioorganic molecules.

### DETAILED DISCUSSION OF THE INVENTION

### I. INTRODUCTION

Significant technological advances in protein chemistry in the last two decades have established mass spectrometry as an indispensable tool for protein study (*Carr et al.,* (1991) "Integration of mass spectrometry in analytical biotechnology," *Anal. Chem.* 63(24):2802-2824; Carr *et al.,* "Overview of Peptide and Protein Analysis by Mass Spectrometry," *Current Protocols in Molecular Biology,* John Wiley & Sons, Inc., New York, unit 10.21, pp. 10.21.1-10.21.27 (1998); Patterson, "Protein Identification and Characterization by Mass Spectrometry," *Current Protocols in Molecular Biology,* John Wiley & Sons, Inc., New York, unit 10.22, pp. 10.22.1-10.22.24 (1998); and Siuzdak, *Mass Spectrometry for Biotechnology,* Academic Press, San Diego (1996)). Mass spectrometry is additionally facilitating the analysis of nucleic acids (Krahmer *et al.,* (2000) "MS for identification of single nucleotide polymorphisms and MS/MS for discrimination of isomeric PCR products," *Anal. Chem.* 72(17):4033-4040 and Koomen *et al.,* (2000) "Improvement of resolution, mass accuracy, and reproducibility in reflected mode DE-MALDI-TOF analysis of DNA using fast evaporation--overlayer sample preparations," *Anal. Chem.* 72(16):3860-3866). Although the resolving power of many chromatographic- and electrophoretic-based separations remains analytically useful, the high sensitivity, speed, and reproducibility of mass spectrometry have boosted its application in all aspects of protein and nucleic acid analysis, including discovery, identification (e.g., peptide mapping, sequencing, *etc.),* quantification and structural characterization.

Analogous to the oligonucleotide chip technologies that allow the study of gene expression profiles, protein biochip technologies have been developed in which proteins are captured on surface features of probes for analysis by mass spectrometry. One such technology takes advantage of surface enhanced laser desorption/ionization time of flight mass spectrometry to facilitate both protein and nucleic acid profiling of complex biologic mixtures. In a version of this technology, affinity mass spectrometry, substrate-bound affinity reagents, either chromatographic or biospecific, capture analytes from a sample. The captured analytes are then desorbed/ionized from the substrate and detected by mass spectrometry. (See, e.g., Hutchens and Yip (1993) "New desorption strategies for the mass spectrometric analysis of macromolecules," *Rapid Commun. Mass Spectrom.* 7:576-580, Kuwata *et al.,* (1998) "Bactericidal domain of lactoferrin: detection, quantitation, and characterization of lactoferricin in serum by SELDI Affinity Mass Spectrometry," *Bioch. Bioph. Res. Comm.* 245:761-773, U.S. Patent No. 5,719,060 to Hutchens and Yip, and WO 98/59360 (Hutchens and Yip)). This innovative technology has numerous advantages over other techniques, such as 2D-PAGE. For example, it is much faster, has higher throughput, requires orders of magnitude lower amounts of sample, has a sensitivity for detecting analyte in the picomole to attomole range, can effectively resolve proteins, nucleic acids and other components having masses in the range of about 2 kDa to about 20 kDa, and is directly applicable to clinical assay development.

The efficacy of the surface enhanced laser desorption/ionization technology, and affinity mass spectrometry, in particular, for discovery and quantification of prostate cancer protein markers in serum, seminal plasma and cell extracts, as well as the development of immunoassays for the detection of known prostate cancer markers has been demonstrated by the inventor and co-workers (Wright *et al.,* (1999) "ProteinChip®-surface enhanced laser desorption/ionization (SELDI) mass spectrometry: A novel protein biochip technology for detection of prostate cancer biomarkers in complex protein mixtures," *Prostate Cancer and Prostatic Diseases,* 2:264-276; Xiao *et al.,* (2000) "Generation of a baculovirus recombinant prostate-specific membrane antigen and its use in the development of a novel protein biochip quantitative immunoassay," *Protein Expr. Purif.,* 19(1):12-21; and Paweletz *et al.,* (2000) "Rapid protein display profiling of cancer progression directly from human tissue using a protein biochip," *Drug Development Research,* 49:34-42). The present invention describes the detection and quantification of PSMA, PSMA', and other prostatic markers in serum samples as well as in other types of samples, and materials and methods for assessing these biomarkers for the diagnosis of prostate cancer or benign prostatic hyperplasia. The invention also provides diagnostic methods involving the detection and quantification of nucleic acids *(e.g.,* mRNA, *etc.)* that encode prostatic markers in cell lysates and other sources. In addition to multiplexed detection/quantification of these protein- and nucleic acid-based markers, the invention also includes biochips, kits, and integrated systems.

### II. CHARACTERIZATION OF MARKERS

The present invention relates to the detection/quantification of the prostatic biomarkers, PSMA and PSMA'. Two general classes of these prostatic markers: (1) protein-based markers, and (2) nucleic acid-based markers. Furthermore, this invention provides multiplex assays in which other biomarkers are detected and/or quantified in addition to PSMA or PSMA'. These other protein-based markers include, e.g., PSA, prostatic acid phosphatase (PAP), prostate specific peptide 94 (PSP94), prostate stem cell antigen (PSCA), or the like. Nucleic acid-based markers typically include nucleic acid sequences, such as mRNAs, which encode the protein-based markers. Amino acid sequences and the mRNA sequences that encode them which relate to these and other markers of interest are readily ascertainable from various public databases including GenBank® and Entrez® Protein database. A description of the biomarkers of interest follows.

### A. Protein-Based Markers

PSMA was initially identified using monoclonal antibody 7E11.C5 (Horoszewicz *et al.,* (1987) "Monoclonal antibodies to a new antigenic marker in epithelial cells and serum of prostatic cancer patients," *Anticancer Res.* 7:927-936) and detected as a 100kDa band in cell lysates and seminal plasma by Western blotting (Feng *et al.,* (1991) "Purification and biochemical characterization of the 7E11-C5 prostate carcinoma-associated antigen," *Proc. AACR* 32 (abs. 1418):239; Troyer *et al.,* (1993) "Molecular characterization of the 7E11-C5 prostate tumor-associated antigen," *J. Urol.* 147 (abs. 482):333A; and *Troyer et al.,* (1995) "Biochemical characterization and mapping of the 7E11-C5.3 epitope of the prostate-specific membrane antigen," *Urologic Oncology* 1:29-37). It has been identified as a 750-amino acid, type II transmembrane glycoprotein that has an unglycosylated molecular weight of about 80-85 kDa (Israeli *et al.,* (1993) "Molecular cloning of a complementary DNA encoding a prostate-specific membrane antigen," *Cancer Res.* 53:227-230 and Israeli *et al.,* U.S. Pat. No. 5,538,866 "PROSTATE-SPECIFIC MEMBRANE ANTIGEN"). Additionally, the gene encoding PSMA has been mapped to chromosome 11 (Rinker-Schaeffer *et al.,* (1995) "Localization and mapping of the prostate specific membrane antigen (PSM) gene to human chromosome 11," *Genomics* 30:105). PSMA is also a glutamate-preferring neurocarboxypeptidase/novel folate hydrolase (Pinto *et al.,* (1996) "Prostate-specific antigen: a novel folate hydrolase in human prostatic carcinoma cells," *Clin. Cancer Res.* 2:11445-11451).

PSMA's expression is increased in association with progression and hormone-refractory prostate cancer (Israeli *et al.,* (1994) "Expression of the prostate-specific membrane antigen," *Cancer Res.* 54:1807-1811). High PSMA expression in prostate tissues with weak expression in brain, salivary glands, and small intestines has been detected using immunohistochemistry, Western blotting and RT-PCR analyses (*Wright et al.,* (1995) "Expression of prostate-specific membrane antigen in normal, benign and malignant prostate tissues," *Urologic Oncology* 1:18-28 and Israeli *et al.,* (1994) *Cancer Res.* 54:1807-1811, *supra).* Several other tissues express the PSMA mRNA but not the protein. PSMA is also expressed on the neovasculature of a variety of human cancers (Liu *et al.,* (1997) "Monoclonal antibodies to the extracellular domain of prostate-specific membrane antigen also react with tumor vascular endothelium," *Cancer Res.* 57:3629-3634).

PSMA' is a truncated species of PSMA that lacks the N-terminal sequence (*i.e.*, the membrane spanning region). *See,* Su *et al.,* (1995) "Alternatively spliced variants of prostate-specific membrane antigen RNA: ratio of expression as a potential measurement of progression," *Cancer Res.* 55:1441-1443. The sequence of amino acids in PSMA' includes amino acids 57-750 in PSMA, and is therefore cytosolic and not detected with the 7E11-C5 antibody. Full-length PSMA was found to be expressed about 10 times higher than the truncated PSMA' in RNA extracted from tumors using an RNase protection assay, about equal expression of both forms in RNA extracted from BPH tissue, and one tenth as much PSMA as PSMA' in RNA extracted from normal prostate tissue (Su *et al.,* (1995) *Cancer Res.* 55:1441-1443, *supra).* Two proteins have been found in LNCaP cell lysates and sera. One protein is full length 100 kDA PSMA, while the other smaller 89 kDa protein is suspected of being PSMA'.

Efforts to determine the clinical utility of PSMA, PSMA' and other prostatic markers as a diagnostic or prognostic biomarkers have been hampered by the lack of a sensitive immunoassay for quantification of those markers in body fluids. The present invention includes an immunoassay to quantify PSMA and other markers that uses a novel ProteinChip® Surface Enhanced Laser Desorption/Ionization (SELDI) mass spectrometry system (Ciphergen Biosystems, Inc., CA). To illustrate, in one embodiment, murine anti-PSMA monoclonal antibody 7E11-C5 is immobilized on proteinG coated ProteinChip® arrays to directly identify and quantify the 100 kDa PSMA in samples using surface enhanced laser desorption/ionization mass spectrometry without the use of a second PSMA antibody or label as is typically used for, e.g., ELISA and other immunoassay formats. The present invention provides the first successful technique for quantifying PSMA in, *e.g.*, serum.

In addition to PSMA and PSMA', this invention provides for multiplex assays of other prostatic biomarkers as well. These include the following markers, without limitation.

The prostate specific antigen is an example of an additional prostatic marker that is optionally quantified according to the diagnostic assays described herein. It is a well-characterized marker for prostate cancer. PSA is biochemically a 33-kDa serine protease that is secreted by the epithelial cells of the prostate. Additional details regarding PSA are described in various publications, including, *e.g.*, Carducci, *et al.,* (1999) "Prostate-specific antigen and other markers of therapeutic response," *Urol. Clin. North Am.* 26(2):291-302; Henkel, (1998) "Prostate cancer," *FDA Consum.* 32(5):22-27; Henry and O'Mahony (1999) "Treatment of prostate cancer," *J. Clin. Pharm. Ther.* 24(2):93-102; and Chu (1997) "Prostate-specific antigen and early detection of prostate cancer," *Tumour Biol.* 18(2):123-134.

Prostatic acid phosphatase is another prostatic marker that is optionally detected and quantified according to the methods described herein. Androgens regulate the expression of both human PAP and PSA, which are both major prostate epithelium-specific differentiation antigens. Specific articles relating to PAP include, e.g., Ahmann and Schifman (1987) "Prospective comparison between serum monoclonal prostate specific antigen and acid phosphatase measurements in metastatic prostatic cancer," *J. Urol.* 137:431-434; Ercole *et al.,* (1987) "Prostate specific antigen and prostatic acid phosphatase in the monitoring and staging of patients with prostatic cancer," *J. Urol.* 138:1181-1184; Gittes (1991) "Carcinoma of the prostate," *N. Eng. J. Med.* 324:236-245; Hetherington *et al.,* (1988) "Contribution of bone scintigraphy, prostatic acid phosphatase and prostate-specific antigen to the monitoring of prostatic cancer," *Eur. Urol.* 14:1-5; and Myers and Grizzle (1997) "Changes in biomarker expression in the development of prostatic adenocarcinoma," *Biotech Histochem.* 72(2):86-95.

The prostatic secretory protein of 94 amino acids is also optionally utilized as a prostatic marker, *e.g.*, in the multiplex embodiments of the present invention. PSP94, also named beta-microseminoprotein, is one of the major proteins secreted by the human prostate. It is a small, nonglycosylated protein, rich in cysteine residues, that was first isolated as a major protein from human seminal plasma. Subsequently, its homologous proteins have been identified, and their cDNAs or genes have been cloned in primates, pigs, and rodents. Additional information pertaining to PSP94 can be found in, *e.g*., Kwong *et al.,* (2000) "PSP94 (or beta-microseminoprotein) is a secretory protein specifically expressed and synthesized in the lateral lobe of the rat prostate," *Prostate* 42(3):219-29 and Chan (1999) "In situ hybridization study of PSP94 (prostatic secretory protein of 94 amino acids) expression in human prostates," *Prostate* 41(2):99-109.

The prostate stem cell antigen is another example of a marker that is optionally detected/quantified in samples via gas phase ion spectrometry. PSCA is a homologue of the Thy-1/Ly-6 family of glycosylphosphatidylinositol (GPI)-anchored cell surface antigens. The gene encoding the antigen maps to chromosome region 8q24.2. PSCA mRNA is expressed in the basal cells of normal prostate and in more than 80% of prostate cancers. Additional details regarding PSCA are described in, *e.g.*, Dannull *et al.,* (2000) "Prostate stem cell antigen is a promising candidate for immunotherapy of advanced prostate cancer," Cancer Res. 60(19):5522-5528; Gu *et al.,* (2000) "Prostate stem cell antigen (PSCA) expression increases with high gleason score, advanced stage and bone metastasis in prostate cancer," *Oncogene* 9(10):1288-1296; and Reiter *et al.,* (2000) "Coamplification of prostate stem cell antigen (PSCA) and MYC in locally advanced prostate cancer," *Genes Chromosomes Cancer* 27(1):95-103.

Essentially any marker is optionally detected and quantified according to the methods described herein. In particular, many other prostatic markers in addition to those described above are also known. Other characteristics of these markers are further described in the example section below.

### B. Nucleic Acid-Based Markers

Virtually any nucleic acid marker of prostate cancer is suitable for analysis as described herein. As such, no attempt is made to identify all of the known nucleic acids. Certain preferred nucleic acid markers of interest include, e.g., mRNAs encoding PSMA, PSMA', PAP, PSP94, PSCA, or the like. Nucleic acids corresponding to these mRNAs, or from which these mRNAs are ascertainable, can be obtained from public databases, such as GenBank. RNAs encoding PSMA and PSMA' are described in, *e.g.,* Su *et al.,* (1995) *Cancer Res.* 55:1441-1443, *supra.* Additional information about the other mRNAs is available assorted publications including, e.g., Zelivianski *et al.,* (1998) "Cloning and analysis of the promoter activity of the human prostatic acid phosphatase gene," *Biochem. Biophys. Res. Commun.* 245(1):108-12; Magklara *et al.,* (2000) "Expression of prostate-specific antigen and human glandular kallikrein 2 in the thyroid gland," *Clin. Chim. Acta* 300(1-2):171-80; Xuan et al., (1995) "Alternative splicing of PSP94 (prostatic secretory protein of 94 amino acids) mRNA in prostate tissue," *Oncogene* 11(6):1041-1047; and Reiter et al., (1998) "Prostate stem cell antigen: a cell surface marker overexpressed in prostate cancer," *Proc. Natl. Acad. Sci. U.S.A.* 95(4):1735-40.

In certain embodiments, PSMA or PSMA' mRNAs are quantified individually in*, e.g.*, cell lysate derived samples. In other embodiments, selected combinations of these and/or other mRNAs in a sample can be detected and quantified simultaneously in a multiplexed format using selected cDNAs which correspond to the selected mRNA combination.

### III. DETECTION AND QUANTIFICATION OF MARKERS

The present invention provides methods for the detection and/or quantification of PSMA and PSMA' by gas phase ion spectrometry and, in particular, by laser desoiption/ionization mass spectrometry. The method involves fractionating a sample that is being tested for the presence of PSMA/PSMA' to at least partially remove other components from the sample, and analyzing the fractionated PSMA/PSMA' by gas phase ion spectrometry. LDI-MS can be traditional MALDI or it can be surface enhanced through the use of a probe whose sample-presenting surface is actively involved in the presentation of the analyte to the energy source.

In one embodiment, the LDI-MS method is surface enhanced through the use of a solid phase-bound adsorbent that captures the analyte from the sample, followed by desorption/ionization of the analyte from the solid phase. In one embodiment this method, the solid-phase bound adsorbent is an antibody that specifically binds PSMA/PSMA'. In another embodiment, the solid phase bound adsorbent is a protein, such as protein G, that specifically binds to an immunoglobulin, e.g., the Fc portion. In this embodiment, the sample is contacted with an antibody that specifically binds PSMA/PSMA'. Then, the solid phase bound adsorbent is used to capture the PSMA/PSMA' from the sample through the antibody to which is it bound. In multiplex assays, the sample can be contacted with antibodies that specifically bind to all of the biomarkers desired for capture. In one embodiment, the solid phase bound adsorbent is provided in the form of a biochip adapted for insertion into the gas phase ion spectrometer. Such a chip can comprise a substrate, such as metal, having a surface that has surface features. Each feature can comprise the adsorbent. In this embodiment, the sample is applied to the surface feature for capture of the desired sample components and unbound components are washed away. Then the biochip is inserted into the gas phase ion spectrometer and the analyte is desorbed/ionized with the energy source and detected.

The markers can be detected in a number of biological samples. The sample is preferably a biological fluid sample. Examples of a biological fluid sample useful in the invention include body fluid, cell lysate, seminal fluid, seminal plasma, prostatic fluid, saliva, blood, lymph, lung/bronchial washes, mucus, feces, nipple secretions, sputum, tears, urine, or the like. Serum is a preferred sample source for certain embodiments of the invention. Cell lysate samples are optionally derived from, e.g., primary tissue or cells, cultured tissue or cells, normal tissue or cells, diseased tissue or cells, benign tissue or cells, cancerous tissue or cells, salivary glandular tissue or cells, intestinal tissue or cells, neural tissue or cells, renal tissue or cells, lymphatic tissue or cells, bladder tissue or cells, urogenital tissues or cells, tumoral tissue or cells, tumoral neovasculature tissue or cells, or the like. In preferred embodiments, a cell lysate sample is derived from prostatic tissue or cells. Biological samples are optionally collected according to any known technique, such as venipuncture, biopsy, or the like. Methods of culturing tissues or cells are described in various publications including, e.g., Ausubel *et al.,* eds., *Current Protocols in Molecular Biology,* a joint venture between Greene Publishing Associates, Inc. and John Wiley & Sons, Inc., New York (supplemented through 1999); Freshney, *Culture of Animal Cells, a Manual of Basic Technique,* 3^{rd} Ed., Wiley-Liss, New York (1994); and Humason, *Animal Tissue Techniques,* 4^{th} Ed., W.H. Freeman and Company, New York (1979), and the references cited therein.

Any suitable method can be used to detect and quantify one or more of the markers described herein. For example, gas phase ion spectrometry can be used. This technique includes, e.g., laser desorption/ionization mass spectrometry. In some embodiments, the sample can be prepared prior to gas phase ion spectrometry, e.g., pre-fractionation, two-dimensional gel chromatography, high performance liquid chromatography, or the like to assist detection of markers. Detection of markers can be achieved using methods other than gas phase ion spectrometry. For example, traditional immunoassays (e.g., ELISA, Western blotting, *etc.)* can be used to detect the markers in a sample. These detection methods are described in detail below.

### A. Detection by Gas Phase Ion Spectrometry

In a preferred embodiment, markers present in a sample are detected using gas phase ion spectrometry, and more preferably, using mass spectrometry. In one embodiment, matrix-assisted laser desorption/ionization ("MALDI") mass spectrometry can be used. In MALDI, the sample is typically quasi-purified to obtain a fraction that essentially consists of a marker or markers using, e.g., protein separation methods such as two-dimensional gel electrophoresis or high performance liquid chromatography (HPLC). Additional details relating to MALDI are included in, e.g., Skoog *et al., Principles of Instrumental Analysis, 5^{th}* Ed., Harcourt Brace & Co., Philadelphia (1998) and Siuzdak, *Mass Spectrometry for Biotechnology, supra.*

In another embodiment, surface-enhanced laser desorption/ionization mass spectrometry ("SELDI") can be used. Surface enhanced laser desorption/ionization uses a substrate comprising adsorbents to capture markers, which can then be directly desorbed and ionized from the substrate surface during mass spectrometry. Since the substrate surface in surface enhanced laser desorption/ionization captures markers, a sample need not be quasi-purified as in MALDI. However, depending on the complexity of a sample and the type of adsorbents used, it may be desirable to prepare a sample to reduce its complexity prior to surface enhanced laser desorption/ionization analysis.

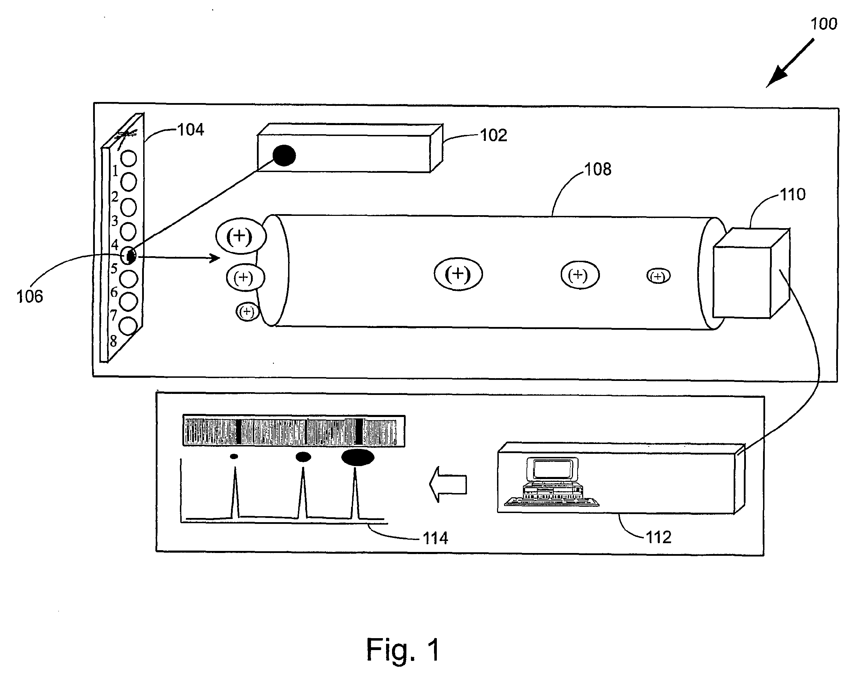

Figure 1 schematically depicts integrated surface enhanced laser desorption/ionization-TOF-MS system **100.** As shown, photon energy produced by laser source **102** impacts biochip **104** at surface feature **106,** which includes a selected adsorbent with captured markers. The photon energy causes captured markers at surface feature **106** to desorb and ionize. The desorbed ions are then accelerated through flight tube/mass analyzer **108.** Ions are separated according to mass/charge ratios, which as depicted is simply the mass of the ionic species, because each ion is singly charged. As further illustrated, smaller ions travel faster than larger ions, thereby resolving the species according to mass. Ions produce a detectable signal at detector **110** which signal is processed by data analysis workstation **112** to generate mass spectrum **114.** Integrated systems are described further below.

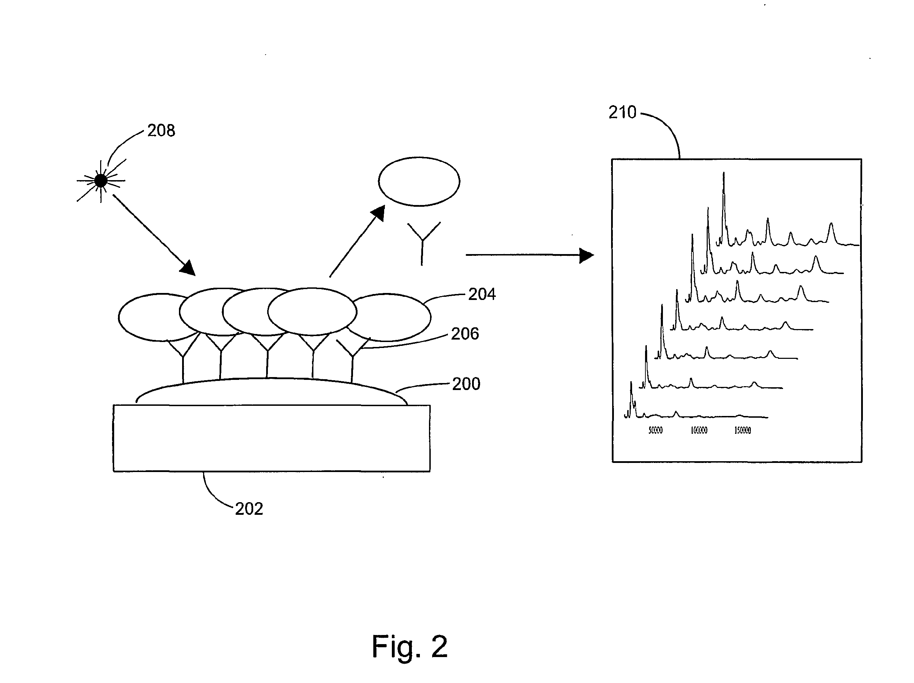

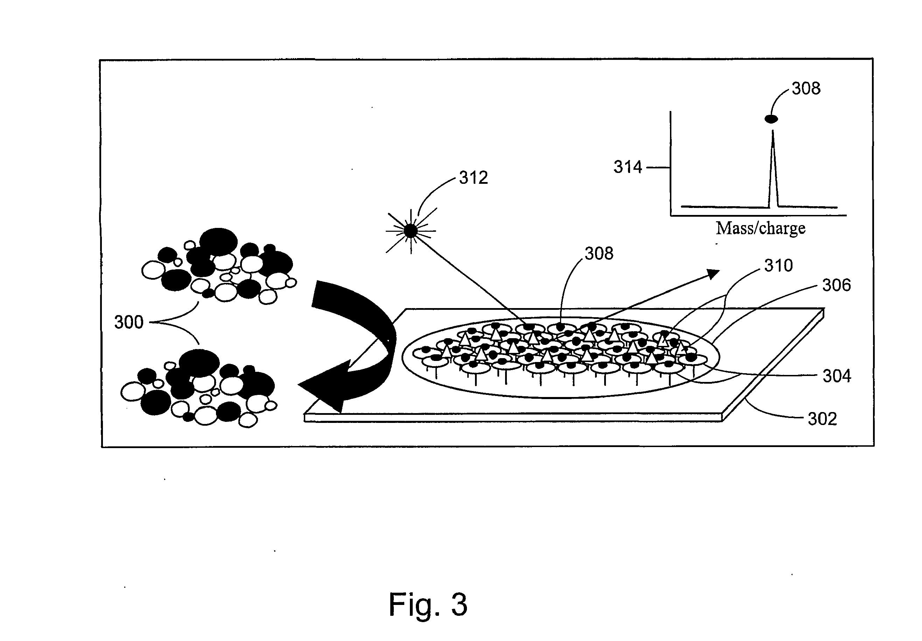

Figure 2 schematically shows a PSMA surface enhanced laser desorption/ionization immunoassay which includes immobilized protein G **200** on biochip **202.** As additionally shown, captured PSMA **204** is retained by antibody adsorbent **206** which is bound to immobilized protein G **200.** Incident photon energy from laser **208** causes the desorption and ionization of adsorbent and PSMA, which is then detected in a TOF-mass spectrometer to produce mass spectra **210.** Figure 3 also schematically illustrates certain aspects of the surface enhanced laser desorption/ionization-TOF-MS technology. As depicted, sample **300** is applied to biochip **302** which includes adsorbent **304** bound to surface feature **306.** Components of sample **300** that are not captured by adsorbent **304** are optionally washed away (*e.g.,* eluted or the like) from biochip **304** prior to mass analysis. In other embodiments, sample **300** is analyzed directly without being washed. Following capture (*e.g.*, affinity capture or the like) of markers **308** in sample **300,** energy absorbing molecules **310** are added to biochip **302** to absorb energy from ionization source **312** (*i.e.,* a laser) to aid desorption of markers **308** from the surface of biochip **302.** Mass spectrum **314** is produced by mass spectral analysis of desorbed/ionized markers **308.**

Various sample preparation methods to assist detection of markers in a sample and gas phase ion spectrometry methods are described in detail below.

### 1. Preparation of a Sample Prior to Gas Phase Ion Spectrometry

Optionally, one or combination of methods described below or other methods known in the art can be used to prepare a sample to further assist detection and characterization of markers in a sample. In some embodiments, a sample can be pre-fractionated to provide a less complex sample prior to gas phase ion spectrometry analysis. For example, a serum sample can be pre-fractionated according to size of proteins to reduce complexity of proteins in the sample. Moreover, pre-fractionation protocols can provide additional information regarding physical and chemical characteristics of markers. For example, if a sample was pre-fractionated using an anion-exchange spin column, and if a marker is eluted at a certain pH, this elution characteristic provides information regarding binding properties of the marker. In another example, a sample can be pre-fractionated by removing proteins or other molecules in the sample that are present in a high quantity or that may interfere with the detection of markers in a sample. Other suitable sample preparation protocols will be apparent to one of skill in the art, and they can also be applied in embodiments of the present invention.

### a) Size Exclusion Chromatography

In one embodiment, a sample can be pre-fractionated according to size of, e.g., proteins in a sample using size exclusion chromatography. For a biological sample wherein the amount of sample available is small, preferably a size selection spin column is used. For example, K-30 spin column (Ciphergen Biosystems, Inc.) can be used. In general, the first fraction that is eluted from the column ("fraction 1") has the highest percentage of high molecular weight proteins; fraction 2 has a lower percentage of high molecular weight proteins; fraction 3 has even a lower percentage of high molecular weight proteins; fraction 4 has the lowest amount of large proteins; and so on. Each fraction can then be analyzed by gas phase ion spectrometry for the detection of markers.

### b) Separation of Biomolecules by Gel Electrophoresis

In another embodiment, biomolecules (*e.g.*, proteins, nucleic acids, *etc.)* in a sample can be separated by high-resolution electrophoresis, *e.g.,* one or two-dimensional gel electrophoresis, Northern blotting, or the like. A fraction containing a marker can be isolated and further analyzed by gas phase ion spectrometry. Preferably, two-dimensional gel electrophoresis is used to generate two-dimensional array of spots of biomolecules, including one or more markers. *See, e.g.,* Jungblut and Thiede, *Mass Spectr. Rev.* 16:145-162 (1997).

The two-dimensional gel electrophoresis can be performed using methods known in the art. *See, e.g.,* Deutscher ed., *Methods In Enzymology* vol. 182. Typically, biomolecules in a sample are separated by, *e.g.,* isoelectric focusing, during which biomolecules in a sample are separated in a pH gradient until they reach a spot where their net charge is zero (*i.e*., isoelectric point). This first separation step results in one-dimensional array of biomolecules. The biomolecules in one dimensional array is further separated using a technique generally distinct from that used in the first separation step. For example, in the second dimension, biomolecules separated by isoelectric focusing are further separated using a polyacrylamide gel, such as polyacrylamide gel electrophoresis in the presence of sodium dodecyl sulfate (SDS-PAGE). SDS-PAGE gel allows further separation based on molecular mass of biomolecules. Typically, two-dimensional gel electrophoresis can separate chemically different biomolecules in the molecular mass range from 1000-200,000 Da within complex mixtures.

Biomolecules in the two-dimensional array can be detected using any suitable methods known in the art. For example, biomolecules in a gel can be labeled or stained (*e.g.,* with Coomassie blue or silver staining). If gel electrophoresis generates spots that correspond to the molecular weight of one or more markers of the invention (*e.g.,* 100 kDa PSMA), the spot can be is further analyzed by gas phase ion spectrometry. For example, spots can be excised from the gel and analyzed by gas phase ion spectrometry. Alternatively, the gel containing biomolecules can be transferred to an inert membrane by applying an electric field. Then a spot on the membrane that approximately corresponds to the molecular weight of a marker can be analyzed by gas phase ion spectrometry. In gas phase ion spectrometry, the spots can be analyzed using any suitable techniques, such as MALDI or surface enhanced laser desorption/ionization (*e.g.,* using ProteinChip^{®} array) as described in detail below.

Prior to gas phase ion spectrometry analysis, it may be desirable to cleave biomolecules in the spot into smaller fragments using cleaving reagents, such as proteases *(e.g*., trypsin) or nucleases. The digestion of biomolecules into small fragments provides a mass fingerprint of the biomolecules in the spot, which can be used to determine the identity of markers if desired.

### c) High Performance Liquid Chromatography

In yet another embodiment, high performance liquid chromatography (HPLC) can be used to separate a mixture of biomolecules in a sample based on their different physical properties, such as polarity, charge and size. HPLC instruments typically consist of a reservoir of mobile phase, a pump, an injector, a separation column, and a detector. Biomolecules in a sample are separated by injecting an aliquot of the sample onto the column. Different biomolecules in the mixture pass through the column at different rates due to differences in their partitioning behavior between the mobile liquid phase and the stationary phase. A fraction that corresponds to the molecular weight and/or physical properties of one or more markers can be collected. The fraction can then be analyzed by gas phase ion spectrometry to detect markers. For example, the spots can be analyzed using either MALDI or surface enhanced laser desorption/ionization (*e.g.*, using ProteinChip^{®} array) as described in detail below.

### d) Modification of Marker Before Analysis

Optionally, a marker can be modified before analysis to improve its resolution or to determine its identity. For example, the markers may be subject to proteolytic digestion before analysis. Any protease can be used. Proteases, such as trypsin, that are likely to cleave the markers into a discrete number of fragments are particularly useful. The fragments that result from digestion function as a fingerprint for the markers, thereby enabling their detection indirectly. This is particularly useful where there are markers with similar molecular masses that might be confused for the marker in question. Also, proteolytic fragmentation is useful for high molecular weight markers because smaller markers are more easily resolved by mass spectrometry. If the target marker is a nucleic acid, such as an mRNA, nucleases are optionally used to modify the marker prior to mass spectral analysis. In another example, biomolecules can be modified to improve detection resolution. For instance, neuraminidase can be used to remove terminal sialic acid residues from glycoproteins (*e.g*., PSMA, PSMA', *etc.)* to improve binding to an anionic adsorbent (*e.g.*, cationic exchange ProteinChip^{®} arrays) and to improve detection resolution. In another example, the markers can be modified by the attachment of a tag of particular molecular weight that specifically bind to molecular markers, further distinguishing them. Optionally, after detecting such modified markers, the identity of the markers can be further determined by matching the physical and chemical characteristics of the modified markers in a protein database (e.g., SWISS-PRO).

### 2. Contacting a Sample with a Substrate for Gas Phase Ion Spectrometry Analysis

A sample or a sample (*e.g.,* pre-fractionated) that is prepared as described above can be contacted with a substrate. A substrate can be a probe (*e.g.*, a biochip) that is adapted for use with a gas phase ion spectrometer. In the present invention, biochips typically include a substrate with at least one surface feature having at least one adsorbent bound to the substrate in which the adsorbent is capable of capturing, *e.g.,* PSMA or PSMA', prostatic marker mRNAs, and/or other markers. Alternatively, a substrate can be a separate material that can be placed onto a probe that is adapted for use with a gas phase ion spectrometer. For example, a substrate can be a solid phase, such as a polymeric, paramagnetic, latex or glass bead or resin comprising, e.g., a functional group for binding markers. The substrate can then be positioned onto a probe.

A probe (*e.g.*, a biochip) can be in any suitable shape as long as it is adapted for use with a gas phase ion spectrometer (*e.g.,* removably insertable into a gas phase ion spectrometer). For example, the probe can be in the form of a strip, a plate, or a dish with a series of wells at predetermined addressable locations or have other surfaces features. The probe can also be shaped for use with inlet systems and detectors of a gas phase ion spectrometer. For example, the probe can be adapted for mounting in a horizontally and/or vertically translatable carriage that horizontally and/or vertically moves the probe to a successive position without requiring repositioning of the probe by hand.

The surface features of probes or biochips include various embodiments. For example, a biochip optionally includes a plurality of surface features. For example, the plurality of surface features is arranged in a line, an orthogonal array, a circle, or an n-sided polygon, wherein n is three or greater. The plurality of surface features typically includes a logical or spatial array. Optionally, each of the plurality of surface features comprises identical or different adsorbents, or one or more combinations thereof. For example, at least two of the plurality of surface features optionally include identical or different adsorbents, or one or more combinations thereof. In certain embodiments, the biochip further includes at least one other adsorbent in addition to the adsorbent capable of capturing PSMA or PSMA' in which the other adsorbent is bound to the substrate at one or more of the plurality of surface features. The other adsorbent is typically capable of capturing at least one other prostatic marker, such as PSMA, PSMA', PSA, free-PSA, complexed-PSA, PAP, PSP94, PSCA, or the like. Suitable adsorbents are described in greater detail below.

The probe substrate can be made of any suitable material. For example, the probe substrate material can include, but is not limited to, insulating materials (*e.g.,* glass such as silicon oxide, plastic, ceramic), a magnetic material, semi-conducting materials (*e.g.*, silicon wafers), or electrically conducting materials (*e.g.*, metals, such as nickel, brass, steel, aluminum, gold, metalloids, alloys or electrically conductive polymers), polymers, organic polymers, conductive polymers, biopolymers, native biopolymers, metal coated with organic polymers, or any combinations thereof. The probe substrate material can also be solid or porous. Probes suitable for use in embodiments of the invention are described in, e.g., U.S. Patent No. 5,617,060 (Hutchens and Yip) and WO 98/59360 (Hutchens and Yip).

### a) Analysis of Samples on an Inert Substrate

If complexity of a sample has been substantially reduced using the preparation methods described above, the sample can be contacted with any suitable substrate for gas phase ion spectrometry. For example, the substrate surface can be inert and need not comprise adsorbents for binding markers, since further separation of other biomolecules from markers is not necessary. In some embodiments, preferably a sample is prepared by two-dimensional gel electrophoresis or HPLC to obtain a fraction that contains markers prior to contacting the fraction with a substrate. Then the markers in the spot or fraction can be resolved using gas phase ion spectrometry (e.g., traditional MALDI) without further fractionation, or using other known methods in the art.

Prior to gas phase ions spectrometry analysis, an energy absorbing molecule ("EAM") or a matrix material is typically applied to markers on the substrate surface. The energy absorbing molecules can assist absorption of energy from an energy source from a gas phase ion spectrometer, and can assist desorption of markers from the probe surface. Exemplary energy absorbing molecules include cinnamic acid derivatives, sinapinic acid ("SPA"), cyano hydroxy cinnamic acid ("CHCA") and dihydroxybenzoic acid. Other suitable energy absorbing molecules are known to those skilled in the art. *See, e.g.,* U.S. Patent 5,719,060 (Hutchens & Yip) for additional description of energy absorbing molecules.

The energy absorbing molecule and the sample containing markers can be contacted in any suitable manner. For example, an energy absorbing molecule is mixed with a sample containing markers, and the mixture is placed on the substrate surface, as in traditional MALDI process. In another example, an energy absorbing molecule can be placed on the substrate surface prior to contacting the substrate surface with a sample. In another example, a sample can be placed on the substrate surface prior to contacting the substrate surface with an energy absorbing molecule. Then the markers can be desorbed, ionized and detected as described in detail below.

### b) Analysis of Samples on a Substrate Surface Comprising Adsorbents

In some embodiments, complexity of a sample can be further reduced using a substrate that comprises adsorbents capable of binding one or more markers. Adsorbents need not be biospecific (e.g., biomolecular interaction adsorbents) for markers (e.g., antibodies that specifically bind markers) as long as adsorbents have binding characteristics suitable for binding markers. For example, adsorbents can comprise chromatographic adsorbent, such as a hydrophobic interaction adsorbent or group, a hydrophilic interaction adsorbent or group, a cationic adsorbent or group, an anionic adsorbent or group, a metal-chelating adsorbent or group (e.g., nickel, cobalt, *etc.),* lectin, heparin, or any combination thereof. In other embodiments, the adsorbent includes a biomolecular interaction adsorbent, such as an affinity adsorbent, a polypeptide, an enzyme, a prostatic marker substrate, a receptor, an antibody, or the like. For example, in preferred embodiments, the biomolecular interaction adsorbent includes a monoclonal antibody that specifically captures the PSMA or PSMA' and optionally, other prostatic markers (*e.g.,* PSMA, PSMA', PSA, free-PSA, complexed-PSA, PAP, PSP94, PSCA, or mRNAs encoding these or other prostatic markers).

Examples of various types of adsorbents are well-known in the art. For instance, adsorbents comprising a hydrophobic group include matrices having aliphatic hydrocarbons, *e.g.*, C₁-C₁₈ aliphatic hydrocarbons and matrices having aromatic hydrocarbon functional group such as phenyl groups. Adsorbents comprising a hydrophilic group include, e.g., glass *(e.g.,* silicon oxide), or hydrophilic polymers such as polyethylene glycol, dextran, agarose, or cellulose. Adsorbents comprising a cationic group include, e.g., matrices of secondary, tertiary or quaternary amines. Adsorbents comprising an anionic group include, e.g., matrices of sulfate anions (SO₃⁻) and matrices of carboxylate anions (COO⁻) or phosphate anions (OPO₃⁻). Adsorbents comprising metal chelating groups include, e.g., organic molecules that have one or more electron donor groups which form coordinate covalent bonds with metal ions, such as copper, nickel, cobalt, zinc, iron, and other metal ions such as aluminum and calcium. Adsorbents comprising an antibody include, e.g., an antibody that specifically binds to any one of the markers provided herein. Probes with some of these adsorbents are also commercially available (*e.g.*, Normal Phase ProteinChip^{®} array, SAX2 ProteinChip^{®} array, IMAC3 ProteinChip^{®} array, *etc.,* all available from Ciphergen Biosystems, Inc. (Fremont, CA)). In preferred embodiments, adsorbents are substantially similar to or the same as the adsorbents which were initially used to enrich and identify the markers from a urine sample *(e.g.*, SAX2 ProteinChip^{®} array).

In embodiments of the invention that include the detection/quantification of mRNAs in a sample, the selected adsorbents typically include DNAs (*e.g.,* cDNAs) corresponding to the targeted prostatic marker mRNAs. Nucleic acid adsorbents are optionally synthesized or purchased from commercial sources. For example, oligo- or polynucleotide sequences for use as adsorbents can readily be synthesized by techniques well-known to those of skill. Furthermore, essentially any nucleic acid is optionally custom ordered from any of a variety of commercial sources, such as The Midland Certified Reagent Company (mcrc@oligos.com), The Great American Gene Company (www.genco.com), ExpressGen, Inc. (www.expressgen.com), Operon Technologies, Inc. (www.operon.com), and many others.

Searchable sequence information is available, e.g., from various public nucleic acid databases, such as GenBank. For example, PSMA and/or PSMA' adsorbents can be designed based upon sequences corresponding to exemplary accession numbers, such as NM_006102, AF119386, AF007544, AF061571, S76978, AF254358, AF254357, AW620338, AW473087, AF107214, NM_004476, AW278819, AW277437, AW168915, AW000926, AI872104, AI791154, AI768508, AI766427, AJ007318, AI690667, AI672408, AI648702, AI478207, AI474492, AI424589, AI356718, AI094658, AI050871, AF016832, AF016830, AF016829, AF016828, AF016827, AF016826, AF011896, AF007413, AF007412, AF00741, AF006604, AA897668, AA879028 AF044684, AA652917, AA435800, AA631303, AF008934, AA371450, AA370337, N75691, N64840, N64840,123813,123812, 123811, 123810, 123809,123808,123807,123806,123805,123804,123803,123802,123801,123800, 123799,123798,123797,123796,123795,123794, and/or M99487. These sequences generally correspond to partial or full-length nucleic acid sequences (*e.g.*, mRNAs, cDNAs, genes, *etc.*) relating to PSMA and PSMA' which can be used alone, or in combination, to ascertain appropriate sequences for use as PSMA or PSMA' adsorbents. Similar sequence information is also available for other prostatic markers including, e.g., PSA, PAP, PSP94, PSCA, or the like.

The probes can be produced using any suitable methods depending on the selection of substrate materials and/or adsorbents. For example, a metal surface can be coated with silicon oxide, titanium oxide or gold, and the coated surface can be derivatized with, *e.g.*, a bifunctional linker to bind and attach an adsorbent. For example, one end of a bifunctional linker can covalently bind with a functional group on the surface and the other end can be further derivatized with groups that function as an adsorbent. In another example, a porous silicon surface generated from crystalline silicon can be chemically modified to include adsorbents for binding markers. In another example, adsorbents can be formed directly on the substrate surface by *in situ* polymerizing a monomer solution which comprises, *e.g.*, substituted acrylamide monomers, substituted acrylate monomers, or derivatives thereof comprising a functional group of choice as an adsorbent. The polymerization of the monomer solution can provide hydrogel adsorbents with a greater capacity for binding biomolecules. The deposition of nucleic acid-based adsorbents (e.g., DNA, cDNAs, *etc.)* is described further in, *e.g.,* U.S. Pat. No. 5,807,522 "METHODS FOR FABRICATING MICROARRAYS OF BIOLOGICAL SAMPLES," to Brown *et al*., and Pease *et al.,* (1994) "Light-generated oligonucleotide arrays for rapid DNA sequence analysis," *Proc. Natl. Acad. Sci. U.S.A.*, 91:5022-5026.

Adsorbents that bind the markers can be applied to the substrate in any suitable pattern (*e.g.*, continuous or discontinuous). For example, one or more adsorbents can be present on the substrate surface. If multiple types of adsorbents are used, the substrate surface can be coated such that one or more binding characteristics vary in one or two-dimensional gradient. If discontinuous, plural adsorbents can be on the substrate surface in predetermined addressable locations. The addressable locations can be arranged in any pattern, but are preferably in a regular pattern, such as lines, orthogonal arrays, or regular curves (e.g., circles). For example, a probe can comprise discontinuous spots of adsorbents. Each addressable location may comprise the same or different adsorbent. The spots are "addressable" in that during mass spectrometry, an energy source, such as a laser, is directed to, or "addresses" each spot to desorb and ionize markers.

A sample is contacted with a substrate comprising an adsorbent in any suitable manner, e.g., bathing, soaking, dipping, spraying, washing over, or pipetting, *etc.* Generally, a volume of sample containing from a few attomoles to 100 picomoles of marker in about 1 µl to 500 µl is sufficient for binding to the adsorbent. The sample can contact the probe substrate comprising an adsorbent for a period of time sufficient to allow the marker to bind to the adsorbent. Typically, the sample and the substrate comprising the adsorbent are contacted for a period of between about 30 seconds and about 12 hours, and preferably, between about 30 seconds and about 15 minutes. Typically, the sample is contacted to the probe substrate under ambient temperature and pressure conditions. For some samples, however, modified temperature (typically 4°C through 37°C) and pressure conditions can be desirable, which conditions are determinable by those skilled in the art.

After the substrate contacts the sample or sample solution, it is preferred that unbound materials on the substrate surface are washed out so that only the bound materials remain on the substrate surface. Washing a substrate surface can be accomplished by, *e.g.,* bathing, soaking, dipping, rinsing, spraying, or washing the substrate surface with an eluant. A microfluidics process is preferably used when an eluant is introduced to small spots of adsorbents on the probe. Typically, the eluant can be at a temperature of between 0°C and 100°C, preferably between 4°C and 37°C. In some embodiments, washing unbound materials from the probe surface may not be necessary if markers bound on the probe surface can be resolved by gas phase ion spectrometry without a wash.

Any suitable eluants *(e.g.,* organic or aqueous) can be used to wash the substrate surface. Preferably, an aqueous solution is used. Exemplary aqueous solutions include a HEPES buffer, a Tris buffer, or a phosphate buffered saline, *etc.* To increase the wash stringency of the buffers, additives can be incorporated into the buffers. These include, but are limited to, ionic interaction modifier (both ionic strength and pH), water structure modifier, hydrophobic interaction modifier, chaotropic reagents, affinity interaction displacers. Specific examples of these additives can be found in, *e.g.,* PCT publication WO98/59360 (Hutchens and Yip). The selection of a particular eluant or eluant additives is dependent on other experimental conditions (*e.g.*, types of adsorbents used or markers to be detected), and can be determined by those of skill in the art.

Prior to desorption and ionization of biomolecules including markers from the probe surface, an energy absorbing molecule ("EAM") or a matrix material is typically applied to markers on the substrate surface. The types of EAM and the methods for applying EAM is discussed above, and will not repeated in this section.

### 3. Desorption/Ionization and Detection

Markers on the substrate surface can be desorbed and ionized using gas phase ion spectrometry. Any suitable gas phase ion spectrometers can be used as long as it allows markers on the substrate to be resolved. Preferably, gas phase ion spectrometers allow quantification of markers.

In one embodiment, a gas phase ion spectrometer is a mass spectrometer. In a typical mass spectrometer, a substrate or a probe comprising markers on its surface is introduced into an inlet system of the mass spectrometer. The markers are then desorbed by a desorption source such as a laser, fast atom bombardment, high energy plasma, electrospray ionization, thermospray ionization, liquid secondary ion MS, field desorption, *etc*. The generated desorbed, volatilized species consist of preformed ions or neutrals which are ionized as a direct consequence of the desorption event. Generated ions are collected by an ion optic assembly, and then a mass analyzer disperses and analyzes the passing ions. The ions exiting the mass analyzer are detected by a detector. The detector then translates information of the detected ions into mass-to-charge ratios. Detection of the presence of markers or other substances will typically involve detection of signal intensity. This, in turn, can reflect the quantity and character of markers bound to the substrate. Any of the components of a mass spectrometer (e.g., a desorption source, a mass analyzer, a detector, *etc.)* can be combined with other suitable components described herein or others known in the art in embodiments of the invention.

Preferably, a laser desorption time-of-flight mass spectrometer is used in embodiments of the invention. In laser desorption mass spectrometry, a substrate or a probe comprising markers is introduced into an inlet system. The markers are desorbed and ionized into the gas phase by laser from the ionization source. The ions generated are collected by an ion optic assembly, and then in a time-of-flight mass analyzer, ions are accelerated through a short high voltage field and let drift into a high vacuum chamber. At the far end of the high vacuum chamber, the accelerated ions strike a sensitive detector surface at a different time. Since the time-of-flight is a function of the mass of the ions, the elapsed time between ion formation and ion detector impact can be used to identify the presence or absence of markers of specific mass to charge ratio.

In another embodiment, an ion mobility spectrometer can be used to detect markers. The principle of ion mobility spectrometry is based on different mobility of ions. Specifically, ions of a sample produced by ionization move at different rates, due to their difference in, e.g., mass, charge, or shape, through a tube under the influence of an electric field. The ions (typically in the form of a current) are registered at the detector which can then be used to identify a marker or other substances in a sample. One advantage of ion mobility spectrometry is that it can operate at atmospheric pressure.

In yet another embodiment, a total ion current measuring device can be used to detect and characterize markers. This device can be used when the substrate has a only a single type of marker. When a single type of marker is on the substrate, the total current generated from the ionized marker reflects the quantity and other characteristics of the marker. The total ion current produced by the marker can then be compared to a control (*e.g*., a total ion current of a known compound). The quantity or other characteristics of the marker can then be determined.

### 4. Analysis of Data

Data generated by desorption and detection of markers can be analyzed using any suitable means. In one embodiment, data is analyzed with the use of a programmable digital computer, e.g., as part of an integrated system. The computer program generally contains a readable medium that stores codes. Certain code can be devoted to memory that includes the location of each feature on a probe, the identity of the adsorbent at that feature and the elution conditions used to wash the adsorbent. The computer also contains code that receives as input, data on the strength of the signal at various molecular masses received from a particular addressable location on the probe. This data can indicate the number of markers detected, including the strength of the signal generated by each marker.

In particular, the invention includes a device or integrated system for quantifying PSMA or PSMA' and optionally, other prostatic markers in selected samples. The device or system typically includes an adsorbent (*e.g.*, a solid phase adsorbent, such as a biochip) capable of capturing, *e.g.,* the PSMA or PSMA' in the sample and a gas phase ion spectrometer for quantifying, *e.g.,* the PSMA or PSMA' captured on the adsorbent. The device or integrated system also typically includes a computer or a computer readable medium, operably connected to the gas phase ion spectrometer, that includes a computer program having one or more of, *e.g.*, (i) an instruction set for analyzing or processing data quantified by the gas phase ion spectrometer, (ii) an instruction set for entering data into a database, or, (iii) an instruction set for determining correlations between at least one quantified prostatic marker, or combinations of quantified prostatic markers, and a diagnosis of prostate cancer, benign prostate hyperplasia, or a negative diagnosis.

Data analysis typically includes the steps of determining signal strength (*e.g.,* height of peaks) of a marker detected and removing "outliers" (data deviating from a predetermined statistical distribution). The observed peaks can be normalized, a process whereby the height of each peak relative to some reference is calculated. For example, a reference can be background noise generated by instrument and chemicals (*e.g*., energy absorbing molecule) which is set as zero in the scale. Then the signal strength detected for each marker or other biomolecules can be displayed in the form of relative intensities in the scale desired (e.g., 100). Alternatively, a standard (*e.g.,* bovine serum albumin) may be admitted with the sample so that a peak from the standard can be used as a reference to calculate relative intensities of the signals observed for each marker or other markers detected.

The computer can transform the resulting data into various formats for displaying. In one format, referred to as "spectrum view or retentate map," a standard spectral view can be displayed, wherein the view depicts the quantity of marker reaching the detector at each particular molecular weight. In another format, referred to as "peak map," only the peak height and mass information are retained from the spectrum view, yielding a cleaner image and enabling markers with nearly identical molecular weights to be more easily seen. In yet another format, referred to as "gel view," each mass from the peak view can be converted into a grayscale image based on the height of each peak, resulting in an appearance similar to bands on electrophoretic gels. In yet another format, referred to as "3-D overlays," several spectra can be overlaid to study subtle changes in relative peak heights. In yet another format, referred to as "difference map view," two or more spectra can be compared, conveniently highlighting unique markers and markers which are up- or downregulated between samples. Marker profiles (spectra) from any two samples may be compared visually. In yet another format, Spotfire Scatter Plot can be used, wherein markers that are detected are plotted as a dot in a plot, wherein one axis of the plot represents the apparent molecular of the markers detected and another axis represents the signal intensity of markers detected. For each sample, markers that are detected and the amount of markers present in the sample can be saved in a computer readable medium. This data can then be compared to a control (*e.g.*, a profile or quantity of markers detected in control, *e.g*., subjects in whom prostate cancer or BPH is undetectable).

### B. Detection by Immunoassay

In another embodiment, an immunoassay can be used to detect and analyze markers (*e.g*., PSMA, PSMA', or the like) in a sample. This method comprises: (a) providing an antibody that specifically binds to a marker; (b) contacting a sample with the antibody; and (c) detecting the presence of a complex of the antibody bound to the marker in the sample.

To prepare an antibody that specifically binds to a marker, purified markers or their nucleic acid sequences can be used. Nucleic acid and amino acid sequences for markers can be obtained by further characterization of these markers. For example, each marker can be peptide mapped with a number of enzymes (*e.g*., trypsin, V8 protease, *etc.).* The molecular weights of digestion fragments from each marker can be used to search the databases, such as SWISS-PRO database, for sequences that will match the molecular weights of digestion fragments generated by various enzymes. Using this method, the nucleic acid and amino acid sequences of other markers can be identified if these markers are known proteins in the databases.

Alternatively, the proteins can be sequenced using protein ladder sequencing. Protein ladders can be generated by, for example, fragmenting the molecules and subjecting-fragments to enzymatic digestion or other methods that sequentially remove a single amino acid from the end of the fragment. Methods of preparing protein ladders are described, for example, in International Publication WO 93/24834 (Chait *et al*.) and United States Patent 5,792,664 (Chait *et al*.). The ladder is then analyzed by mass spectrometry. The difference in the masses of the ladder fragments identify the amino acid removed from the end of the molecule.

If the markers are not known proteins in the databases, nucleic acid and amino acid sequences can be determined with knowledge of even a portion of the amino acid sequence of the marker. For example, degenerate probes can be made based on the N-terminal amino acid sequence of the marker. These probes can then be used to screen a genomic or cDNA library created from a sample from which a marker was initially detected. The positive clones can be identified, amplified, and their recombinant DNA sequences can be subcloned using techniques which are well known. *See, e.g., Current Protocols for Molecular Biology* (Ausubel *et al.,* Green Publishing Assoc. and Wiley-Interscience (supplemented through 1999)) *and Molecular Cloning: A Laboratory Manual,* 2nd Ed. (Sambrook *et al.,* Cold Spring Harbor Laboratory, N.Y. (1989)).

Using the purified markers or their nucleic acid sequences, antibodies that specifically bind to a marker can be prepared using any suitable methods known in the art. *See, e.g.,* Coligan, *Current Protocols in Immunology* (1991); Harlow & Lane, *Antibodies: A Laboratory Manual* (1988); Goding, *Monoclonal Antibodies: Principles and Practice* (2d ed. 1986); and Kohler & Milstein, *Nature* 256:495-497 (1975). Such techniques include, but are not limited to, antibody preparation by selection of antibodies from libraries of recombinant antibodies in phage or similar vectors, as well as preparation of polyclonal and monoclonal antibodies by immunizing rabbits or mice (*see, e.g.,* Huse *et al., Science* 246:1275-1281 (1989); Ward *et al., Nature* 341:544-546 (1989)).

After the antibody is provided, a marker can be detected and/or quantified using any of suitable immunological binding assays known in the art (*see,* e.g., U.S. Patent Nos. 4,366,241; 4,376,110; 4,517,288; and 4,837,168). Useful assays include, for example, an enzyme immune assay (EIA) such as enzyme-linked immunosorbent assay (ELISA), a radioimmune assay (RIA), a Western blot assay, or a slot blot assay. These methods are also described in, *e.g., Methods in Cell Biology: Antibodies in Cell Biology,* volume 37 (Asai, ed. 1993); *Basic and Clinical Immunology* (Stites & Terr, eds., 7th ed. 1991); and Harlow & Lane, *supra.*

Generally, a sample obtained from a subject can be contacted with the antibody that specifically binds the marker. Optionally, the antibody can be fixed to a solid support to facilitate washing and subsequent isolation of the complex, prior to contacting the antibody with a sample. Examples of solid supports include glass or plastic in the form of, e.g., a microtiter plate, a stick, a bead, or a microbead. Antibodies can also be attached to a probe substrate or Protein Chip^{®} array and can be analyzed by gas phase ion spectrometry as described above. The sample is preferably a biological fluid sample taken from a subject. Examples of biological samples include urine, blood, serum, plasma, tears, saliva, tissue, *etc.* In a preferred embodiment, the biological fluid comprises serum. The sample can be diluted with a suitable eluant before contacting the sample to the antibody.

After incubating the sample with antibodies, the mixture is washed and the antibody-marker complex formed can be detected. This can be accomplished by incubating the washed mixture with a detection reagent. This detection reagent may be, *e.g.,* a second antibody which is labeled with a detectable label. Exemplary detectable labels include magnetic beads (*e.g.*, DYNABEADS^{™}), fluorescent dyes, radiolabels, enzymes (*e.g.*, horse radish peroxide, alkaline phosphatase and others commonly used in an ELISA), and colorimetric labels such as colloidal gold or colored glass or plastic beads. Alternatively, the marker in the sample can be detected using an indirect assay, wherein, for example, a second, labeled antibody is used to detect bound marker-specific antibody, and/or in a competition or inhibition assay wherein, for example, a monoclonal antibody which binds to a distinct epitope of the marker is incubated simultaneously with the mixture.

Throughout the assays, incubation and/or washing steps may be required after each combination of reagents. Incubation steps can vary from about 5 seconds to several hours, preferably from about 5 minutes to about 24 hours. However, the incubation time will depend upon the assay format, marker, volume of solution, concentrations and the like. Usually the assays will be carried out at ambient temperature, although they can be conducted over a range of temperatures, such as 10°C to 40°C.

Immunoassays can be used to determine the presence or absence of a marker, such as PSMA or PSMA' in a sample as well as the quantity of a marker in a sample. First, a test amount of a marker in a sample can be detected using the immunoassay methods described above. If a marker is present in the sample, it will form an antibody-marker complex with an antibody that specifically binds the marker under suitable incubation conditions described above. The amount of an antibody-marker complex can be determined by comparing to a standard. A standard can be, e.g., a known compound or another protein known to be present in a sample. As noted above, the test amount of marker need not be measured in absolute units, as long as the unit of measurement can be compared to a control.

The methods for detecting these markers in a sample have many applications. For example, one or more markers can be measured to aid prostate cancer or BPH diagnosis or prognosis. In another example, the methods for detection of the markers can be used to monitor responses in a subject to cancer treatment. In another example, the methods for detecting markers can be used to assay for and to identify compounds that modulate expression of these markers *in vivo* or *in vitro.*

### IV. DIAGNOSIS OF PROSTATE CANCER OR BENIGN PROSTATE HYPERPLASIA

Experiments demonstrate that compared to normal subjects (*e.g.*, having a negative diagnosis for prostate cancer or benign prostate hyperplasia), PSMA and/or PSMA' is up-regulated compared to normal in prostate cancer and downregulated, compared to normal, in benign prostate hyperplasia. Therefore, this invention also provides methods for aiding a prostate cancer or benign prostate hyperplasia diagnosis using the prostatic markers described herein. These markers can be used alone or in combination with other markers. For example, PSMA is typically differentially present in samples (*e.g.*, serum samples) from prostate cancer or BPH patients and normal subjects in whom prostate cancer or BPH is undetectable. Therefore, detection of one or more of the markers described herein in a person would provide useful information regarding the probability that the person has prostate cancer or BPH. As illustrated further in the examples described below, prostate cancer patients may have higher concentrations of serum PSMA than normal patients.

In particular, the invention relates to a method for aiding in a diagnosis of prostate cancer or benign prostate hyperplasia that includes detecting (*e.g.,* by gas phase ion spectrometry) PSMA or PSMA' in a sample from a subject, and correlating the detected PSMA or PSMA' with a probable diagnosis of prostate cancer, benign prostate hyperplasia, or a negative diagnosis. The correlation generally takes into account a presence or absence of the PSMA or PSMA' in the sample and a frequency of PSMA or PSMA' detection in a control. In addition, the correlation also typically takes into account a quantity of the PSMA or PSMA' in the sample relative to a control quantity of the PSMA or PSMA'. In certain embodiments, an immunoassay is used for detecting the PSMA or PSMA' in the sample. In some embodiments, both PSMA and PSMA' are detected and correlated, while in other embodiments, other markers, such as nucleic acids encoding PSMA or other prostatic markers are used. More specifically, detecting an amount of PSMA/PSMA' above the normal range is positively correlated with a diagnosis of prostate cancer and detecting an amount of PSMA/PSMA' below the normal range is positively correlated with a diagnosis of benign prostate hyperplasia.

Any suitable samples can be obtained from a subject to detect markers. Preferably, a sample is a serum sample from the subject. If desired, the sample can be prepared as described above to enhance detectability of the markers. For example, to increase the detectability of markers, such as PSMA, a serum sample from the subject can be optionally fractionated by, e.g., size exclusion chromatography. Sample preparations, such as pre-fractionation protocols, are optional and may not be necessary to enhance detectability of markers depending on the methods of detection used. For example, sample preparation may be unnecessary if antibodies that specifically bind markers are used to detect the presence of markers in a sample.

Any suitable method is optionally used to detect a marker or markers in a sample. For example, gas phase ion spectrometry or a traditional immunoassay (*e.g.,* ELISA, Western blotting) can be used as described above or as known in the art. Using these methods, one or more markers can be detected. Preferably, a sample is tested for the presence of a plurality of markers. Detecting the presence of a plurality of markers, rather than a single marker alone, would provide more information for the diagnostician. Specifically, the detection of a plurality of markers in a sample would increase the percentage of true positive and true negative diagnoses and would decrease the percentage of false positive or false negative diagnoses.

The detection of the marker or markers is then correlated with a probable diagnosis of prostate cancer or BPH. In some embodiments, the detection of the mere presence or absence of a marker, without quantifying the amount of marker, is useful and can be correlated with a probable diagnosis of prostate cancer or BPH. For example, the mere detection of one or more of markers in a subject being tested may indicate that the subject has a higher probability of having a prostate cancer or BPH. In other embodiments, the detection of markers can involve quantifying the markers to correlate the detection of markers with a probable diagnosis of prostate cancer or BPH. For example, some or all of the markers may be present at a higher quantity in serum samples of prostate cancer or BPH patients than in serum samples of normal subjects. Thus, if the amount of the markers detected in a subject being tested is higher compared to a control amount, then the subject being tested may have a higher probability of having a prostate cancer or BPH.

When the markers are quantified, it can be compared to a control. A control can be, e.g., the average or median amount of marker present in comparable samples of normal subjects in whom prostate cancer or BPH is undetectable. The control amount is measured under the same or substantially similar experimental conditions as in measuring the test amount. For example, if a test sample is obtained from a subject's urine sample and a marker is detected using a particular probe, then a control amount of the marker is preferably determined from a serum sample of a patient using the same probe. It is preferred that the control amount of marker is determined based upon a significant number of samples from normal subjects who do not have prostate cancer or BPH so that it reflects variations of the marker amounts in that population.

Data generated by mass spectrometry can then be analyzed by a computer software. The software can comprise code that converts signal from the mass spectrometer into computer readable form. The software also can include code that applies an algorithm to the analysis of the signal to determine whether the signal represents a "peak" in the signal corresponding to a marker of this invention, or other useful markers. The software also can include code that executes an algorithm that compares signal from a test sample to a typical signal characteristic of "normal" and prostate cancer or BPH and determines the closeness of fit between the two signals. Any suitable algorithm can be used, e.g., an artificial intelligence program, a heuristic learning algorithm, or the like. For example, an artificial intelligence program that includes a fuzzy logic instruction set, a cluster analysis instruction set, a neural network, a genetic algorithm, or the like, can be used. *See*, *e.g*., Qureshi e*t al., J. Urol.,* 163: 630-633 (2000); Snow, *et al., J. Urol.,* 152:1923 (1994). The software can also include code indicating which the test sample is closest to, thereby providing a probable diagnosis.

### V. KITS

The methods of the present invention may employ a kit for aiding a differential diagnosis of prostate cancer or BPH. For example, the kits can be used to detect any one or more of the markers described herein, which markers are differentially present in samples of a prostate cancer or BPH patient and normal subjects. The kits of the invention have many applications. For example, the kits can be used to differentiate if a subject has prostate cancer or BPH or has a negative diagnosis, thus aiding a prostate cancer or BPH diagnosis. In another example, the kits can be used to identify compounds that modulate expression of one or more of the markers in *in vitro* or *in vivo* animal models for prostate cancer or BPH.

In one embodiment, a kit includes (a) at least one adsorbent that captures PSMA or PSMA', (b) a set of instructions for capturing PSMA or PSMA' from a sample by exposing the sample to the adsorbent and quantifying the captured PSMA or PSMA' by gas phase ion spectrometry, and (c) at least one container for packaging the adsorbent and the set of instructions. Optionally, the kit also includes at least one eluant for washing the adsorbent to remove material other than the captured PSMA or PSMA'. The adsorbent typically includes a solid phase adsorbent. In one embodiment, the solid phase adsorbent is provided as a biochip that includes a substrate with at least one surface feature having the solid phase adsorbent bound.to the substrate. The substrate is generally a probe adapted for use with a gas phase ion spectrometer. The kit optionally includes the probe.

In certain embodiments, the probe includes a substrate with a plurality of surface features. For example, each of the plurality of surface features optionally includes one or more adsorbents bound to the substrate. The plurality of surface features is generally arranged in a line, an orthogonal array, a circle, or an n-sided polygon, wherein n is three or greater. Optionally, the plurality of surface features includes a logical or spatial array. In other embodiments, the solid phase adsorbent includes a bead or resin derivatized with the adsorbent. For example, the bead or resin derivatized with the at least one adsorbent is typically suitable for being placed on a probe adapted for use with a gas phase ion spectrometer. The kit also optionally includes the probe. As an additional option, the kit also includes at least one reference or control. In yet another embodiment, the kit may further comprise a pre-fractionation spin column (*e.g*., K-30 size exclusion column).

The kits may include various types of adsorbents. For example, in some embodiments, the adsorbent includes a chromatographic adsorbent, such as an anionic adsorbent, a cationic adsorbent, a hydrophobic interaction adsorbent, a hydrophilic interaction adsorbent (*e.g.,* silicon oxide, *etc.*), a metal-chelating adsorbent (*e.g.*, nickel, cobalt, *etc.*) or the like. In other embodiments, the adsorbent includes a biomolecular interaction adsorbent, such as an affinity adsorbent, a polypeptide, an enzyme, a prostatic marker substrate, a receptor, an antibody, or the like. In preferred embodiments, the biomolecular interaction adsorbent includes a monoclonal antibody that specifically captures the PSMA or PSMA'. In still other embodiments, the kit further includes at least one other adsorbent in addition to the adsorbent that captures PSMA or PSMA'. The other adsorbent is generally capable of capturing at least one other prostatic marker, such as PSMA, PSMA', PSA, free-PSA, complexed-PSA, PAP, PSP94, PSCA, or the like. As an additional option, the kit also includes (1) an eluant in which the PSMA or PSMA' or at least one other prostatic marker is retained on the adsorbent when washed with the eluant, or (2) instructions to wash the adsorbent with the eluant after contacting the adsorbent with a sample.

In other embodiments of the invention, the kit includes (a) at least one adsorbent that captures a prostatic marker mRNA, (b) a set of instructions for capturing the prostatic marker mRNA from a sample by exposing the sample to the adsorbent and quantifying the captured prostatic marker mRNA by gas phase ion spectrometry, and (c) at least one container for packaging the adsorbent and the set of instructions.

Optionally, the kit can further comprise instructions for suitable operational parameters in the form of a label or a separate insert. For example, the kit may have standard instructions informing a consumer how to wash the probe after, e.g., a serum sample is contacted on the probe. In another example, the kit may have instructions for pre-fractionating a sample to reduce complexity of proteins or nucleic acids in the sample.

In another embodiment, a kit comprises (a) an antibody that specifically binds to a marker; and (b) a detection reagent. Such kits can be prepared from the materials described above, and the previous discussion regarding the materials (e.g., antibodies, detection reagents, immobilized supports, *etc.)* is fully applicable to this section and will not be repeated. Optionally, the kit may further comprise pre-fractionation spin columns. In some embodiments, the kit may further comprise instructions for suitable operation parameters in the form of a label or a separate insert.

Optionally, the kit may further comprise a standard or control information so that the test sample can be compared with the control information standard to determine if the test amount of a marker detected in a sample is a diagnostic amount consistent with a diagnosis of prostate cancer or BPH. For example, a standard can be bovine insulin, bovine serum albumin, *etc*.

### VI. EXAMPLES

The following non-limiting examples are offered only by way of illustration.

### A. Overview

Efforts to determine the clinical utility of PSMA as a diagnostic or prognostic biomarker have been hampered by the lack of a sensitive immunoassay for quantification of PSMA in body fluids. An objective of these studies was to develop a PSMA immunoassay using a novel ProteinChip® technology from Ciphergen Biosystems, Inc. (Fremont, CA), which employs surface enhanced laser desorption/ionization time-of-flight mass spectrometry. In one aspect, murine anti-PSMA monoclonal antibody (MoAb) 7E11-C5.3 was immobilized on protein G coated biochip arrays, samples added, and the chips subjected to surface enhanced laser desorption/ionization mass spectrometry. The 100kDa PSMA was correctly identified directly without the need for a second PSMA antibody or labels as required for ELISA and other immunoassay formats. A linear standard curve (R²=0.985) was generated using increasing concentration (0.5-50ng/µl) of purified recombinant PSMA (rPSMA). *See,* Figure 4. Quantification of PSMA in serum and seminal plasma at nanomolar levels was determined by normalized mass signal integral. A multiplex version of the surface enhanced laser desorption/ionization immunoassay, where two MoAbs (one to PSMA and the other to PSA) were bound to the same biochip array, demonstrated the versatility of the system for rapid and simultaneous quantification of both prostate cancer markers. Preliminary surface enhanced laser desorption/ionization immunoassay results have shown better discrimination between normal donors and prostate cancer patients then by, *e.g.,* Western analyses, and between cancer and benign prostate conditions; both being statistically significant (p<0.001). Prior to these studies, immunoblot and sandwich immunoassay had been used to detect PSMA in body fluids, with uncertain reliability, especially in serum. The surface enhanced laser desorption/ionization immunoassay offers a reliable system to quantify PSMA or PSMA' and to address their viability as a diagnostic/prognostic biomarker for prostate cancer. Furthermore, the surface enhanced laser desorption/ionization immunoassay provides an innovative platform to rapidly and simultaneously measure multiple biomarkers.

### B. Recombinant Prostate Specific Membrane Antigen Protein

The procedure for the generation and purification of a recombinant prostate specific membrane antigen protein was described in detail in Xiao *et al.,* (2000) "Generation of a baculovirus recombinant prostate-specific membrane antigen and its use in the development of a novel protein biochip quantitative immunoassay," *Protein Expression and Purification* 19:12-21. The purified rPSMA protein was quantified by the BCA protein assay (Pierce Chemical, Rockford, IL), and stored at -20°C until use.

### C. Patients and Serum Samples

All the serum samples used in these studies were obtained from the serum bank of the Virginia Prostate Center at Eastern Virginia Medical School, and were the same group of samples that had been used previously to analyze the serum level of PSMA by Western blotting (Beckett *et al.,* (1999) "Prostate-specific membrane antigen (PSMA) levels in sera from healthy men and patients with benign prostate hyperplasia or prostate cancer," *Clin. Cancer Res.* 5:4034-4040). The normal male population was defined by negative digital rectal examination and PSA <4.0ng/ml. The range of age for normal males under the age of 50 was from 25-39 with an average of 34, whereas the range for the normal males over 50 was from 51-73 with an average of 60. Benign prostate hyperplasia patients were those who had bladder outlet obstructive symptoms, increased PSA levels (>4ng/ml) and revealed BPH upon transrectal ultrasound biopsy. No treatment had been received by these patients at the time of serum collection. The prostatitis population constituted patients with chronic symptom complex attributed to prostatic source and were absent of other prostatic pathology including obstruction, infection by urine culture, abscess, or carcinoma. Sera of prostate carcinoma (PCA) were collected from patients with clinical stages of T1 and T2.

### D. Surface Enhanced Laser Desorption/Ionization Immunoassay for PSMA

An immunoassay was developed for the detection and quantification of PSMA in serum using the surface enhanced laser desorption/ionization ProteinChip® technology. The following modification was made based on the method described in Xiao *et al.,* (2000) *Protein Expression and Purification* 19:12-21, *supra.* After coating spots with 1µg of protein G, residual active sites were blocked by incubating the entire biochip in 1M ethanolamine pH 8.0 for 30 minutes (min) with agitation. The biochip was subsequently washed 2x5min with 0.5% Triton X-100 (Sigma) in PBS, and 2x5min with PBS. MoAb 7E11 or 107-1A4 (1.5µg) was added to the spots and incubated at room temperature for 3 hours. Unbound antibodies were washed off by incubating the biochip 2x5min in 0.1 % Triton X-100 in PBS, and 2x5min in PBS. A 96-well bioprocessor was assembled over the biochips, creating multiple sample holders in a format of 96-well plate. To generate a standard curve, 30µl of diluted rPSMA (at 0.5, 1 and 3ng/µl in 0.1% Triton X-100 in PBS) was applied to the spots. *See,* Figure 4. To detect PSMA in serum, 400µl of serum samples (diluted 1:1 with 0.5% Triton X-100 in PBS) were applied to the spots. The samples were incubated overnight at 4°C with vigorous agitation. The biochip was subsequently washed 2x5min with 0.1% Triton X-100 in PBS, 2x5min with PBS, and briefly with HPLC H₂O. Sinapinic acid (Sigma) saturated in 50% (v/v) acetonitrile, 0.5% (v/v) trifluoroacetic acid and 0.05% Triton X-100 was used as the matrix solution, along with β-galactoglobulin (MW 18,363.3 Da) added as an internal standard (IS) for normalization. The matrix solution (2x0.5µl) was applied to the spots, and the chip was air dried at room temperature. The detection of serum PSMA was performed using a PBS-1 mass spectrometer (Ciphergen Biosystems, Inc., CA) with a laser intensity of 60. The data in each spectrum was averaged from 120 laser shots and a total of three spectra were collected per sample. The normalized peak area ratios of PSMA and the internal standard were calculated for both serum PSMA and rPSMA. *See,* Figure 4. The levels of serum PSMA were determined by comparing the serum PSMA/IS peak area ratios to rPSMA/IS area ratios of the standard curve.

### E. Results

### 1. The Determination of Serum PSMA Levels

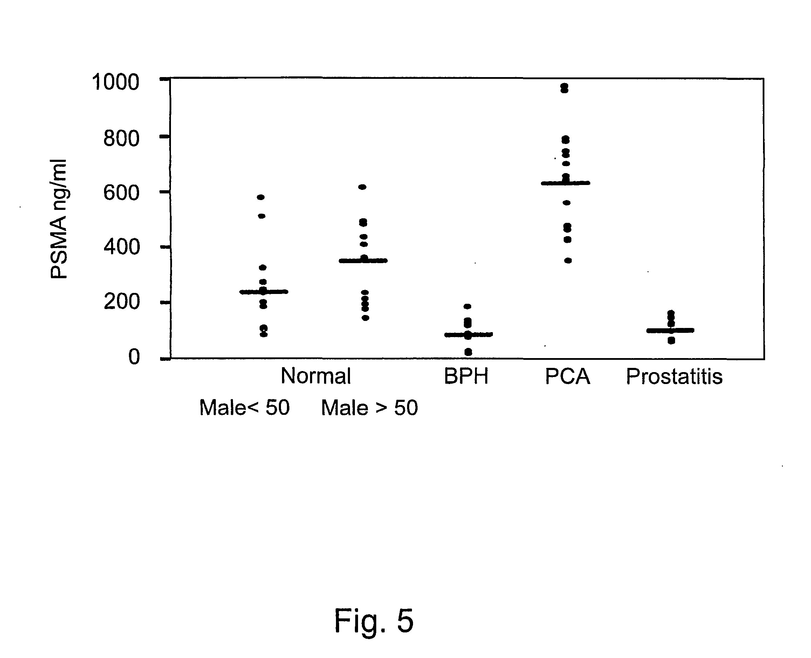

To test the feasibility of using surface enhanced laser desorption/ionization immunoassay for quantifying serum PSMA, a preliminary study was performed on a total of 60 serum samples. These included 24 normal males (12 with age <50; 12 with age >50), 10 BPH, 17 PCA (stage T1 and T2), and 9 prostatitis. The results of these studies are summarized in Figure 5 and Table 1. The level of serum PSMA was found to be low in patients with BPH (mean 117.1ng/ml) and prostatitis (mean 119.9ng/ml), medium in normal males (mean 272.9ng/ml and 359.4ng/ml in age <50 and >50 populations, respectively), and high in patients with PCA (mean 623.1ng/ml). Coefficient of variation (CV%) values included a range of 1.2-20.1 and an average of 10.1.

Statistical analysis (i.e., Student T-test) showed that the average serum PSMA level of normal males under 50 years of age did not differ significantly from the values obtained for normal males >50 (p=0.16), whereas the differences were significant when comparing the average PSMA levels of either normal population versus PCA (p<0.001), normal versus BPH (p<0.001), and BPH versus PCA (p<0.001). In addition, the mean level of serum PSMA in prostatitis patients differed from that of normal and PCA groups (p<0.001), but not from BPH group (p=0.95). Since a similar trend of differences were observed among normal, BPH and PCA populations in previous Western blot analysis (Beckett *et al.,* (1999) *Clin. Cancer Res.* 5:4034-4040, *supra),* this preliminary study indicated the specificity and feasibility of the surface enhanced laser desorption/ionization immunoassay for detection and relative quantification of PSMA in sera.

**TABLE 1**

| | **Normal Male <50** | **Normal Male >50** | **BPH** | **PCA** | **Prostatitis** |
|---|---|---|---|---|---|
| **Number of samples** | 12 | 12 | 10 | 17 | 9 |
| **range (ng/ml)** | 106.4-576.9 | 159.8-611.5 | 35.6-193.8 | 349.5-946.6 | 73.4-168.2 |
| **mean (ng/ml)** | 272.85 | 359.39 | 117.09 | 623.11 | 119.91 |
| | | | | | |
| **p value (normal <50)*** | -- | 0.16 | < 0.001 | < 0.001 | < 0.01 |
| **p value (normal >50)**** | -- | -- | < 0.001 | < 0.001 | < 0.001 |
| **p value (BPH)***** | -- | -- | -- | < 0.001 | 0.95 |
| **p value (CaP)****** | -- | -- | -- | -- | < 0.001 |

| | | | | | |
|---|---|---|---|---|---|
| * p value: normal male <50 v.s. each of the other groups ** p value: normal male >50 v.s. each of the other groups *** p value: BPH v.s. each of the other groups **** p value: PCA v.s. each of the other groups | | | | | |

### 2. The Detection of Putative PSMA Isoforms

Previous studies have reported the existence of a PSMA protein isoform (i.e., PSMA' or PSM') in LNCaP cell lysate, and this isoform can only be detected by antibodies specific to the extracellular domain of PSMA, but not by antibodies to the intracellular domain, such as 7E11 (Grauer *et al.,* (1998) "Identification, purification, subcellular localization of prostate-specific membrane antigen PSM' protein in the LNCaP prostatic carcinoma cell line," *Cancer Res.* 58:4787-4789). MoAb 107-1A4, a MoAb recognizing the extracellular domain of PSMA, was used. Using this MoAb, the 100 kDa full-length PSMA plus an extra peak at 89 kDa was detected in LNCaP cell lysates, as shown in Figure 6A. This smaller protein was not detected by 7E11. The 89 kDa protein is potentially the PSMA' isoform. However, since PSMA' was previously identified with a mass of 95 kDa by Western blot (Grauer *et al.,* (1998) *Cancer Res.* 58:4787-47893, *supra),* the 89 kDa peak will need to isolated and sequenced to determine if in fact it is a PSMA isoform.

To determine the best preactivated chip chemistry for the PSMA surface enhanced laser desorption/ionization immunoassay, LNCaP lysates were used as the antigen source to generate a standard curve for both proteins. Two different preactivated chip surfaces, PS-1 and PS-2 (Ciphergen Biosystems, Inc., Fremont, CA), were compared using MoAbs, 7E11 and 107. As shown in Figure 6B, the PS-1 surface had an overall better performance in capturing the PSMA protein than the PS-2 surface. In case of MoAb 7E11, little of the 100 kDa PSMA (indicated by the arrow) was captured on the PS-2 surface, regardless of the concentration of LNCaP cell lysate. On the other hand, the peak area and intensity of PSMA captured by 7E11 on PS-1 surface was correlated with the concentration of LNCaP cell lysate, suggesting an enhanced specificity and efficiency in antigen recovery. When MoAb 107 was used, both the 89 kDa (arrow at lower of the two indicated mass/charge positions) and the 100 kDa (arrow at higher of the two indicated mass/charge positions) proteins were captured on both surfaces. However, the PS-1 surface showed a better separation and linearity of the two closely existing peaks than the PS-2 surface. Although not shown, a standard curve for both proteins was generated successfully using LNCaP lysate as the antigen source in combination with MoAb 107. Assay optimization may include the use of MoAb 107 with a PBS-II (Ciphergen Biosystems, Inc., CA) and a mass spectrometry grade SPA. The optimized assay will be used to quantify both PSMA and the 89 kDa protein in serum and compare the results with serum PSA levels. Initial results indicate the 100 kDa and 89 kDa proteins are present in serum and seminal plasma in differing amounts (data not shown). Additional studies will also be conducted to quantify PSMA in seminal plasma and urine.

### 3. Multiplex Surface Enhanced Laser Desorption/Ionization Immunoassay for PSMA and PSA

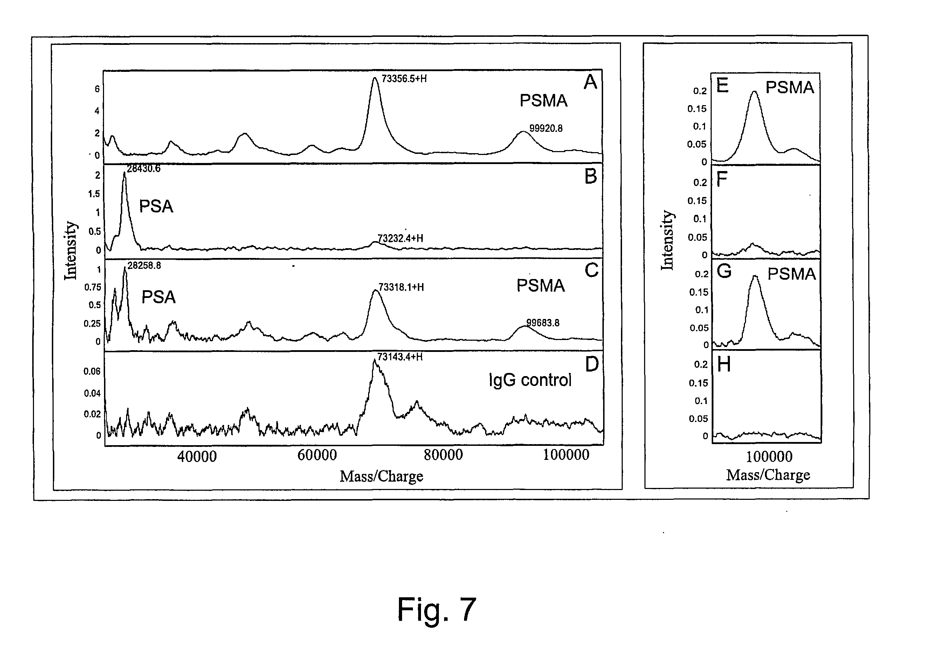

Multiplex assays are relatively easy to develop using the surface enhanced laser desorption/ionization immunoassay system. For example, Figures 7AH show a multiplex immunoassay for detection of PSMA and PSA in seminal plasma. MoAbs to PSA and PSMA or combinations of two antibodies were bound to the preactivated (PS-1) surface after protein G binding. Panel A shows a peak corresponding to PSMA (99,920.8) was identified using the anti-PSMA (7E11-C5.3) MoAb alone. Panel B shows the detection of free PSA (28,430 Da) using the anti-PSA antibody alone. Panel C shows the detection of both free PSA and PSMA with a surface enhanced laser desorption/ionization immunoassay using a single chip array containing MoAbs to both PSA and PSMA. Panel D shows that free PSA and PSMA were not detected when using the isotype-matched control immunoglobulin in place of the biomarker specific MoAbs. The doubly charged immunoglobulin (73.1-73.4 kDa) is observed when either the specific MoAb or control IgG was used (panels A-D). Note that broad peak obtained for PSMA shown in panels A-D, when using the multiplex assay, could be enhanced by selecting the optimal intensity for PSMA and adjusting the mass axis, as shown in panels E-H. This analysis is described further in Wright *et al.,* ProteinChip® surface enhanced laser desorption/ionization (SELDI) mass spectrometry: a novel protein chip technology for detection of prostate cancer biomarkers in complex protein mixtures," *Prostate Cancer and Prostatic Diseases,* 2:264-276. Essentially any combination of markers is optionally detected according to these multiplex immunoassay formats including, e.g., the simultaneous detection/quantification of free- and complexed-PSA and PSMA.

## Claims

1. A method for aiding in a differential diagnosis of prostate cancer, benign hyperplasia, or a negative diagnosis, the method comprising:
(a) exposing a sample obtained from a subject to at least one substrate-bound adsorbent that captures PSMA and/or PSMA', thereby capturing the PSMA and/or the PSMA' in the sample;
(b) detecting the captured PSMA and/or the captured PSMA' by gas phase ion spectrometry; and
(c) correlating the detected PSMA and/or the detected PSMA' with a probable diagnosis of prostate cancer, benign prostate hyperplasia, or a negative diagnosis; wherein an amount of the PSMA and/or the PSMA' above a control amount is positively correlated with a positive diagnosis of prostate cancer and an amount of the PSMA and/or the PSMA' below a control amount is positively correlated with a positive diagnosis of benign prostate hyperplasia.

2. The method of claim 1, further comprising detecting at least one other prostatic marker in the sample, wherein (a) further comprises exposing the sample to at least one substrate-bound adsorbent that captures the other prostatic marker.

3. The method as claimed in claim 1, wherein the correlation takes into account a quantity of the PSMA and/or the PSMA', or a ratio thereof, in the sample relative to a control quantity of the PSMA and/or the PSMA', or a ratio thereof.

4. A method of aiding in a differential diagnosis of prostrate cancer, benign prostate hyperplasia, or a negative diagnosis the method comprising:
(a) exposing a sample obtained from a subject to at least one adsorbent that captures a prostatic marker mRNA, thereby capturing the prostatic marker mRNA in the sample;
(b) detecting the captured prostatic marker mRNA by gas phase ion spectrometry; and,
(c) correlating the detected prostatic marker mRNA with a probable diagnosis of prostate cancer, benign prostate hyperplasia, or a negative diagnosis.

## Patentansprüche

1. Verfahren zur Unterstützung bei der Differentialdiagnose von Prostatakrebs, benigner Hyperplasie oder einer negativen Diagnose, wobei das Verfahren Folgendes umfasst:
(a) das Aussetzen einer von einem Individuum erhaltenen Probe gegenüber zumindest einem substratgebundenen Adsorbens, das PSMA und/oder PSMA' einfängt, wodurch das PSMA und/oder das PSMA' in der Probe eingefangen wird;
(b) das Detektieren des eingefangenen PSMA und/oder des eingefangenen PSMA' durch Gasphasenionenspektrometrie; und
(c) das Korrelieren des detektieren PSMA und/oder des detektierten PSMA' mit einer wahrscheinlichen Diagnose von Prostatakrebs, benigner Prostatahyperplasie oder einer negativen Diagnose; worin eine PSMA- und/oder PSMA'-Menge über einer Kontrollmenge positiv mit einer positiven Diagnose von Prostatakrebs korreliert wird und eine PSMA- und/oder PSMA'-Menge unter einer Kontrollmenge positiv mit einer positiven Diagnose von benigner Prostatahyperplasie korreliert wird.

2. Verfahren nach Anspruch 1, weiters das Detektieren zumindest eines anderen Prostatamarkers in der Probe umfassend, worin (a) weiters das Aussetzen der Probe gegenüber zumindest einem substratgebundenen Adsorbens umfasst, das den anderen Prostatamarker einfängt.

3. Verfahren nach Anspruch 1, worin bei der Korrelation eine PSMA- und/oder PSMA'-Menge, oder ein Verhältnis davon, in der Probe in Bezug zu einer Kontrollmenge des PSMA und/oder PSMA', oder eines Verhältnisses davon, berücksichtigt wird.

4. Verfahren zur Unterstützung bei der Differentialdiagnose von Prostatakrebs, benigner Hyperplasie oder einer negativen Diagnose, wobei das Verfahren Folgendes umfasst:
(a) das Aussetzen einer von einem, Individuum erhaltenen Probe gegenüber zumindest einem Adsorbens, das eine Prostatamarker-mRNA einfängt, wodurch die Prostatamarker-mRNA in der Probe eingefangen wird;
(b) das Detektieren der eingefangenen Prostatamarker-mRNA durch Gasphasenionenspektrometrie; und
(c) das Korrelieren der detektieren Prostatamarker-mRNA mit einer wahrscheinlichen Diagnose von Prostatakrebs, benigner Prostatahyperplasie oder einer negativen Diagnose.

## Revendications

1. Méthode pour aider à un diagnostic différentiel d'un cancer de la prostate, d'une hyperplasie bénigne ou d'un diagnostic négatif, la méthode consistant à:
(a) exposer un échantillon obtenu d'un sujet à au moins un adsorbant lié à un substrat qui capture PSMA et/ou PSMA', pour ainsi capturer PSMA et/ou PSMA' dans l'échantillon;
(b) détecter PSMA capturé et/ou PSMA' capturé par spectométrie d'ions en phase gazeuse; et
(c) mettre en corrélation PSMA détecté et/ou PSMA' détecté avec un diagnostic probable d'un cancer de la prostate, une hyperplasie bénigne de la prostate ou un diagnostic négatif; où une quantité de PSMA et/ou PSMA' au-dessus d'une quantité de contrôle est en corrélation positive avec un diagnostic positif de cancer de la prostate et une quantité de PSMA et/ou de PSMA' en dessous d'une quantité de contrôle est en corrélation positive avec un diagnostic positif d'hyperplasie bénigne de la prostate.

2. Méthode de la revendication 1 consistant de plus à détecter au moins un autre marqueur de la prostate dans un échantillon, où (a) comprend de plus l'exposition de l'échantillon à au moins un adsorbant lié au substrat qui capture l'autre marqueur de la prostate.

3. Méthode de la revendication 1 où la corrélation tient compte d'une quantité de PSMA et/ou PSMA' ou son rapport dans l'échantillon relativement à une quantité de contrôle de PSMA et/ou PSMA' ou son rapport.

4. Méthode aidant au diagnostic différentiel d'un cancer de la prostate, d'une hyperplasie bénigne de la prostate ou d'un diagnostic négatif, la méthode consistant à:
(a) exposer un échantillon obtenu d'un sujet à au moins un adsorbant qui capture un marqueur de l'ARNm de la prostate, pour ainsi capturer le marqueur le l'ARNm de la prostate dans l'échantillon;
(b) détecter l'ARNm du marqueur capturé de la prostate par spectométrie d'ions en phase gazeuse et
(c) mettre en corrélation l'ARNm du marqueur détecté de la prostate avec un diagnostic probable de cancer de la prostate, une hyperplasie bénigne de la prostate ou un diagnostic négatif.
